# EUROPEAN PATENT SPECIFICATION

(11) **EP 2 438 047 B1**
(45) Date of publication and mention of the grant of the patent: **30.07.2014**
(21) Application number: 10784012.6
(22) Date of filing: 02.06.2010
(51) Int. Cl.: C07D 259/00, C07D 405/02, A61K 31/549, A61P 37/02, C07D 405/04, C07D 405/06

(54) **ANTAGONISM OF HUMAN FORMYL PEPTIDE RECEPTOR FOR TREATMENT OF DISEASE**
ANTAGONISMUS EINES MENSCHLICHEN FORMYL-PEPTIDREZEPTORS ZUR BEHANDLUNG EINER KRANKHEIT
ANTAGONISME DU RÉCEPTEUR DU PEPTIDE FORMYL HUMAIN DANS LE TRAITEMENT DE MALADIES

(30) Priority: 22.07.2009 US 227642 P; 02.06.2009 US 183368 P; 02.06.2009 US 183375 P; 22.07.2009 US 227657 P; 02.06.2009 US 183358 P; 22.07.2009 US 227643 P
(43) Date of publication of application: 11.04.2012
(73) Proprietor: Nikan Pharmaceuticals, LLC, West Roxbury, MA 02132 (US)
(72) Inventor: BENSON, John, D., West Roxbury MA 02132 (US)
(74) Representative: Geary, Stephen
(86) International application number: PCT/US2010/037068
(87) International publication number: WO 2010/141584

(56) References cited:
- WO-A1-2006/063470
- WO-A2-02/069902
- WO-A2-03/030834
- US-A1- 2003 104 992
- US-A1- 2006 008 891
- JOHANNES D. AEBI ET AL: "synthesis, conformation, and immunosuppressive activities of three analogues of cyclosporin A modified in the 1-position", J. MED. CHEM., vol. 33, 1990, pages 999-1009,

## Description

### Background of Invention

Eosinophils and neutrophils are blood cells that play an important role in the immune response. Eosinophils are immune cells that, in addition to their role in fighting parasitic infection, are associated with a variety of immune and allergic disorders. They develop in the bone marrow and are mobilized into the blood. Unstimulated circulating eosinophils persist in the bloodstream for 8-12 hours. Eosinophil differentiation is regulated by a number of factors, including IL-3, IL-5, and GM-CSF. In particular, animal models first suggested a predominant role of IL-5 in expansion and mobilization of eosinophils from bone marrow to the bloodstream and to the lung.

Asthma is a highly prevalent allergic disorder of complex and poorly understood etiology. Although a number of animal (e.g. mouse) models exist, none accurately capture all the physiological and pathological features of the disease. Nonetheless, it is clear that eosinophils are among the implicated immune effector cells; this observation consistent with the increased number of eosinophils in airway mucosa of patients suffering from either atopic or allergen associated asthma. Indeed, clinical trials have been undertaken with antibodies that antagonize IL-5 signaling. In several such studies, administration of such antibodies has resulted in a significant and persistent decrease in circulating eosinophils. Moreover, remodeling of the lung tissue is a predominant feature in asthma patients. Such remodeling is thought to chronically abrogate the responsiveness of the airway in asthma patients. Although the exact etiology of such remodeling is not well understood, it is widely believed that chronic localized activity of eosinphils plays an important role.

Hypereosinophilic syndrome (HES) is characterized by persistent elevated levels of eosinophils, leading to respiratory, cardiac, skin and gastrointestinal problems. Eosinophil accumulation in the gastrointestinal tract has also been observed in a number of eosinophil-associated gastrointestinal disorders (EGIDs), including eosinophilic gastroenteritis, allergic colitis, eosinophilis esophagitis, inflammatory bowel disease, and gastrointestinal reflux disease. In each case, localized infiltration of eosinophils has been suggested to play a role in the etiology or pathophysiology of the disease or disorder (reviewed by Hogan and Rothenberg, 2006).

Reslizumab, a humanized monoclonal antibody against IL-5 is currently in clinical trials for treatment of eosinophilic esophagitis and asthma. Clinical efficacy has been demonstrated in several studies examining the use of reslizumab in treatment of eosinophilic esophagitis. Moreover, mepolizumab, another humanized monoclonal antibody directed against IL-5 has been shown to be effective in treatment of HES, and is now being developed as an orphan drug for this indication.

However, there still remains a need for a treatment of eosinophil-associated diseases and disorders, such as allergic disorders, gastrointestinal disorders, or immune disorders.

Neutrophils play a crucial role in defense against infection by being among the first cells that migrate to sites of infection. They are abundant in blood, but absent from normal tissue. Upon chemotactic recruitment to a site of infection and activation through various signaling molecule gradients that stimulate cellular receptors at nanomolar concentrations (e.g. chemoattractants, interleukins, chemokines), neutrophil activation results in degranulation, releasing a battery of proteolytic enzymes and oxidative products, including ozone and hydrogen peroxide. While these events represent important physiological components of innate immune response, several pathological conditions are associated with inappropriate or exaggerated activation of neutrophils and thereby cause excessive tissue damage.

Exaggerated or inappropriate neutrophil response may be detrimental and plays a key role in the etiology, pathophysiology, or symptomology of a number of medically important diseases and disorders, many of which have few if any desirable or effective treatment options. Neutrophil response is regulated by a number of endogenous and exogenous factors. In each case, however, a balance must exist that allows robust enough response to respond to infection, but not reaching a point of unnecessary or undesirable destruction or damage to host tissue. Moreover, the inflammatory response should be self-attenuating, resolving when the insult or infectious organism has been eliminated. Such attenuation is critical to the health of the tissue in question and to the healing process associated with localized tissue damage and associated with infection and immune response.

Cystic fibrosis (CF) is the most common hereditary disorder in Caucasians. Airway inflammation is a major factor in its pathogenesis, with tissue destruction of the lung the greatest cause of morbidity and mortality. Chronic *Pseudomonas aeruginosa* lung infections are also characteristic of the disease; the lungs of almost all CF patients are colonized by this organism before three years of age. Such infections in CF patients elicit massive neutrophil response that is unaccompanied by bacterial clearance. Antibiotic therapy typically has limited efficacy that diminishes over time.

Treatment of CF at present is mostly symptomatic, consisting of antibiotic therapy to combat bacterial infections, physiotherapy to remove the viscous mucous from the airways, the administration of pancreatic enzymes to compensate for loss pf pancreatic function (found in at least 85% of patients), the administration of mucolytic agents, and dietary regulations to optimize energy uptake. Short-term high-dose ibuprofen or other anti-inflammatory agents can be useful in addressing the profound and protracted inflammation characteristic of the CF lung, but toxicity to the liver and other organs (especially in pediatric patients) prevents their long term use. Similarly, corticosteroid therapies have some utility, but side effects preclude their widespread or common use. The efficacy of anti-inflammatory drugs in CF patients does, however, validate the desirability and utility of addressing inflammation as a key mode of effective therapy.

There remains a need for a treatment of neutrophil-associated diseases and disorders, such as CF, COPD and emphysema.

### Summary of the Invention

There remains a need for new treatments and therapies for eosinophil-associated and neutrophil-associated diseases and disorders. There is also a need for compounds useful in the treatment or prevention or amelioration of one or more symptoms of allergic disorders, gastrointestinal disorders, immune disorders, inflammation, CF, COPD and emphysema. Furthermore, there is a need for methods for modulating the activity of eosinophils and neutrophils.

Thus, in one aspect, the invention provides a cyclosporine H derivative of Formula I. In one embodiment, the cyclosporine H derivative has an IC₅₀ of at least 1µM in a radionucleotide binding competition or other suitable quantitative formyl peptide receptor binding assay.

In another aspect, the invention provides a method of treating a subject suffering from an eosinophil-associated disease, comprising administering a therapeutically effective amount of a formyl peptide receptor (FPR) antagonist, such that said subject is treated. The FPR antagonist can be an immunobinder or a nucleic acid aptamer. The immunobinder can also be an anti-FPR antibody.

In another aspect, provided herein is a method of treating a subject suffering from an eosinophil-associated disease, comprising administering a therapeutically effective amount of the cyclosporine H derivative of Formula I, such that said subject is treated. In still another aspect, provided herein is a method of treating a subject suffering from an eosinophil-associated disease, comprising administering a therapeutically effective amount of cyclosporine H or dihydro cyclosporine H, such that said subject is treated.

The eosinophil-associated disease can be an immune disorder, such as eczema, dermatitis, or psoriasis, or an allergic disorder, such as asthma, hay fever, hypereosinophilic syndrome, or an eosinophil-associated gastrointestinal disorder. Non-limiting examples of allergic disorders include eosinophilic gastroenteritis, allergic colitis, eosinophilic esophagitis, inflammatory bowel disease and gastrointestinal reflux disease. It is understood that in the case of respiratory disorders such treatment would possibly also result in changes (i.e. inhibition or delay) of airway remodeling in treated patients. As such, all aspects and embodiments of FPR-inhibitor-mediated treatment disclosed herein would possibly include, but not be limited to attenuation or delay of airway remodeling.

In another aspect, provided herein is a method of inhibiting the activity of formyl peptide receptor in a subject, comprising administering to said subject an effective amount of a formyl peptide receptor (FPR) antagonist, such that eosinophil mobilization in said subject is inhibited. In one embodiment, the FPR antagonist is an immunobinder or a nucleic acid aptamer. In another embodiment, the immunobinder is an anti-FPR antibody.

In another aspect, provided herein is a method for inhibiting the activity of a formyl peptide receptor in a subject, comprising administering to said subject an effective amount of a cyclosporine H derivative of Formula I, such that eosinophil mobilization in said subject is inhibited.

In still another aspect, provided herein is a method for inhibiting the activity of a formyl peptide receptor in a subject, comprising administering to said subject an effective amount of cyclosporine H or dihydro cyclosporine H, such that eosinophil mobilization in said subject is inhibited.

In yet another aspect, provided herein is a method of decreasing eosinophil mobilization in a subject, comprising administering to said subject an effective amount of a formyl peptide receptor (FPR) antagonist, such that eosinophil mobilization in said subject is decreased. In one embodiment, the FPR antagonist is an immunobinder or a nucleic acid aptamer. In another embodiment, the immunobinder is an anti-FPR antibody.

In another aspect, provided herein is a method for decreasing eosinophil mobilization in a subject, comprising administering to said subject an effective amount of the cyclosporine H derivative of Formula I, such that eosinophil mobilization in said subject is decreased. In one embodiment, the cell is a bone marrow cell.

In still another aspect, provided herein is a method for decreasing eosinophil mobilization in a subject, comprising administering to said subject an effective amount of cyclosporine H or dihydro cyclosporine H, such that eosinophil mobilization in said subject is decreased. In one embodiment, the cell is a bone marrow cell.

In another aspect, provided herein is a method of decreasing the level of circulating eosinophils in a subject, comprising administering to said subject a formyl peptide receptor (FPR) antagonist in an amount sufficient to decrease said level of circulating eosinophils in said subject. In one embodiment, the FPR antagonist is an immunobinder or a nucleic acid aptamer. In another embodiment, the immunobinder is an anti-FPR antibody.

In still another aspect, provided herein is a method for decreasing the level of circulating eosinophils in a subject, comprising administering to said subject a cyclosporine H derivative of Formula I in an amount sufficient to decrease said level of circulating eosinophils in said subject.

In yet another aspect, provided herein is a method for decreasing the level of circulating eosinophils in a subject, comprising administering to said subject cyclosporine H or dihydro cyclosporine H in an amount sufficient to decrease said level of circulating eosinophils in said subject. The level of circulating eosinophils can be less than the amount required for diagnosing said subject with hypereosinophilic syndrome.

In another aspect, provided herein is a method for decreasing the accumulation or activation of eosinophils at sites associated with a disease related to increased eosinophilic function or decreasing localized eosinophil cell numbers in a subject, comprising administering to said subject a therapeutically effective amount of a formyl peptide receptor (FPR) antagonist, such that the accumulation or activation of eosinophils at sites associated with a disease related to increased eosinophilic function or localized eosinophil cell numbers in said subject is decreased. In one embodiment, the FPR antagonist is an immunobinder or a nucleic acid aptamer. In another embodiment, the immunobinder is an anti-FPR antibody.

In another aspect, provided herein is a method for decreasing the accumulation of eosinophils at sites associated with a disease related to increased eosinophilic function or decreasing localized eosinophil cell numbers in a subject, comprising administering to said subject a therapeutically effective amount of the cyclosporine H derivative of Formula I, such that the accumulation of eosinophils at sites associated with a disease related to increased eosinophilic function or localized eosinophil cell numbers in said subject is decreased. The site can be, for example, the gastrointestinal tract, the lung or the skin. In one embodiment, the disease related to increased eosinophilic function or localized eosinophil cell numbers is selected from eosinophilic gastroenteritis, allergic colitis, eosinophilis esophagitis, inflammatory bowel disease, and gastrointestinal reflux disease. In another embodiment, the disease related to increased eosinophilic function or localized eosinophil cell numbers is selected from dermatitis, eczema, and psoriasis.

In another aspect, provided herein is a method for decreasing the accumulation of eosinophils at sites associated with a disease related to increased eosinophilic function or decreasing localized eosinophil cell numbers in a subject, comprising administering to said subject a therapeutically effective amount of cyclosporine H or dihydro cyclosporine H, such that the accumulation of eosinophils at sites associated with a disease related to increased eosinophilic function or localized eosinophil cell numbers in said subject is decreased. The site can be, for example, the gastrointestinal tract, the lung or the skin. In one embodiment, the disease related to increased eosinophilic function or localized eosinophil cell numbers is selected from asthma, including, allergen associated, idiopathic, and atopic asthma or chronic obstructive pulmonary disease (COPD), including emphysema and chronic obstructive bronchitis. In another embodiment, the disease related to increased eosinophilic function or localized eosinophil cell numbers is selected from dermatitis, eczema, and psoriasis.

In another embodiment, the cyclosporine H derivative of Formula I, cyclosporine H, or dihydro cyclosporine H is administered in combination with a systemic or localized steroid.

In another embodiment, the cyclosporine H derivative of Formula I, cyclosporine H or dihydro cyclosporine H is administered in combination with an antibody that antagonizes IL-5 signaling, such as reslizumab or mepolizumab.

In another aspect, provided herein is a method of treating a subject suffering from a chronic obstructive pulmonary disease (COPD), comprising administering a therapeutically effective amount of a formyl peptide receptor (FPR) antagonist, such that said subject is treated. In one embodiment, the FPR antagonist is CsH or dihydroCsH. In another embodiment, the FPR antagonist is an immunobinder (*e.g*., an an anti-FPR antibody) or a nucleic acid aptamer.

In another aspect, provided herein is a method of treating a subject suffering from a chronic obstructive pulmonary disease (COPD), comprising administering a therapeutically effective amount of cyclosporine H, dihydro cyclosporine H, or pharmaceutically acceptable salts and solvates thereof, such that said subject is treated.

In still another aspect, provided herein is a method of treating a subject suffering from a chronic obstructive pulmonary disease (COPD), comprising administering a therapeutically effective amount of a cyclosporine H derivative of formula I. In one embodiment, the COPD is selected from the group consisting of chronic bronchitis, chronic obstructive bronchitis, and emphysema, or a combination thereof.

In yet another aspect, provided herein is a method of treating a subject suffering from emphysema, comprising administering a therapeutically effective amount of a formyl peptide receptor (FPR) antagonist, such that said subject is treated. In one embodiment, the FPR antagonist is an immunobinder (*e.g*., an anti-FPR antibody) or a nucleic acid aptamer.

In another aspect, provided herein is a method of treating a subject suffering from emphysema, comprising administering a therapeutically effective amount of cyclosporine H or dihydro cyclosporine H, such that said subject is treated.

In another aspect, provided herein is a method of treating a subject suffering from emphysema, comprising administering a therapeutically effective amount of a cyclosporine H derivative of formula I.

In another aspect, provided herein is a method of treating a subject suffering from an inflammatory disease, comprising administering a therapeutically effective amount of a formyl peptide receptor (FPR) antagonist, such that said subject is treated. In one embodiment, the FPR antagonist is dihydrocyclosporine H (dihydroCsH). In another embodiment, the FPR antagonist is an immunobinder (*e.g*., an anti-FPR antibody) or a nucleic acid aptamer.

In another aspect, provided herein is a method of treating a subject suffering from an inflammatory disease, comprising administering a therapeutically effective amount of dihydro cyclosporine H, or pharmaceutically acceptable salts and solvates thereof, such that said subject is treated.

In another aspect, provided herein is a method of treating a subject suffering from an inflammatory disease, comprising administering a therapeutically effective amount of a cyclosporine H derivative of formula I.

In one embodiment, the inflammatory disease to be treated is selected from the group consisting of acute lung inflammation, chronic lung inflammation, COPD, emphysema, inflammation of the gastrointestinal tract, acute skin inflammation, atopic dermatitis, eczema, neutrophilic dermatosis, rosacea, psoriasis, peritonitis, ischemic reperfusion injury, neutrophil disorder, cystic fibrosis, acne, and diseases caused by or associated with detrimental, exaggerated, or inappropriate neutrophil activation, or a combination thereof.

In one embodiment, the inflammation of the gastrointestinal tract is inflammatory bowel disease (IBD) or Crohn's disease. This disorder to be treated can also be selected from the group consisting of chronic granulomatous disease, Chédiak-Higashi syndrome (CHS), leukocyte adhesion deficiency, hyper IgE syndrome (Jobs syndrome), and other neutrophil-mediated inflammation. In another embodiment, the disease to be treated is cystic fibrosis, inflammatory bowel disease (IBD) or Crohn's disease.

In another aspect, provided herein is a method of treating any of the aforementioned inflammatory diseases comprising administering a therapeutically effective amount of dihydrocyclosporine H, or pharmaceutically acceptable salts and solvates thereof, such that said subject is treated.

### Brief Description of the Drawings

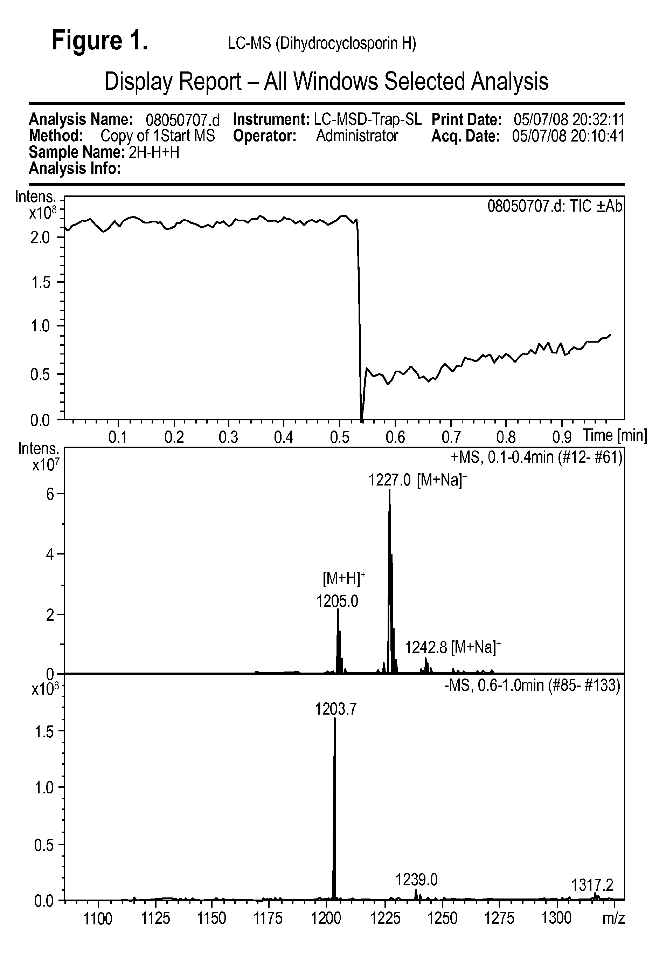

**Figure 1** is an LC-MS spectrum of dihydrocyclosporine H.

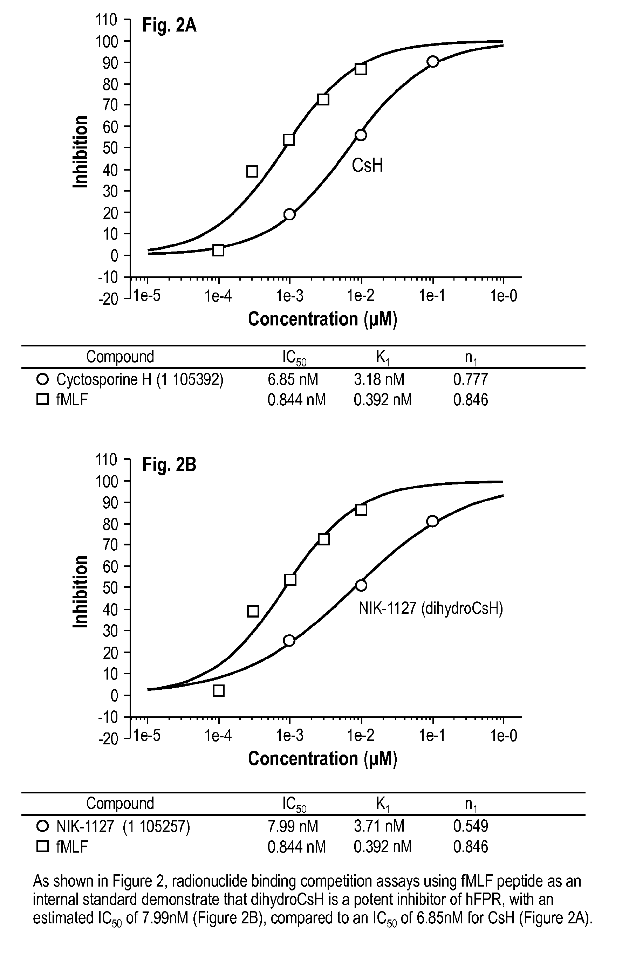

**Figure 2** shows the results of radionuclide binding competition assays using fMLF peptide as an internal standard demonstrate that dihydroCsH is a potent inhibitor of hFPR, with an estimated IC50 of 7.99nM (Figure 2B), compared to an IC50 of 6.85nM for CsH (Figure 2A).

### Detailed Description of Invention

Cyclosporine A, widely known as inhibitor of the T-cell activation via the calcineurin/ cyclophilin pathway is known from WO2006/063470.

Cyclosporine H (CsH) is a known specific antagonist of the formyl peptide receptor (Wenzel-Seifert et al., 1991; Wenzel-Seifert and Seifert, 1993). Provided herein is the therapeutic use of FPR antagonists for the treatment of the eosinophil-associated and neutrophil-associated diseases and disorders, including those enumerated above. In preferred embodiments, such antagonists would include CsH and its derivatives that act as potent and specific FPR antagonists (*e.g*., dihydro CsH and compounds of Formula I). Cyclosporines of this invention are administered at doses resulting in the reduction of circulating eosinophils or neutrophils, or decrease in eosinophils or neutrophils at sites associated with diseases of increased eosinophil or neutrophil function or localized cell number, or resulting in a decrease in activation of eosinophils or neutrophils at such sites.

### Compounds of the Invention

In one aspect, the invention provides a cyclosporine H derivative of the Formula I: 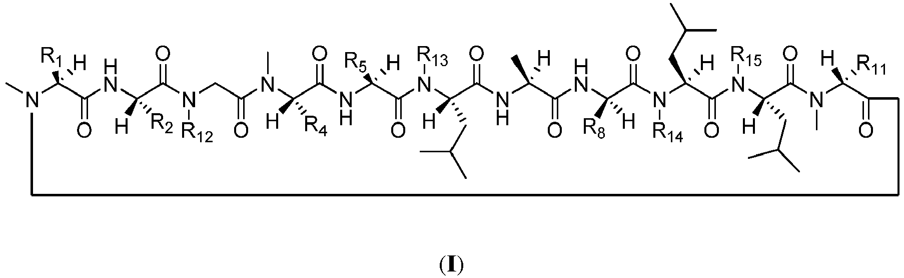 and pharmaceutically acceptable salts and solvates thereof, wherein
R¹ is 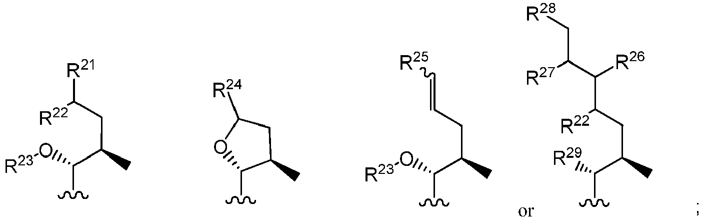
R² is C₁-C₆ alkyl, C₃-C₆ cycloalkyl, or hydroxy-C₁-C₆ alkyl;
R⁴, R⁵ and R¹¹ are independently C₁-C₆ alkyl or C₃-C₆ cycloalkyl;
R⁸ is C₁-C₆ alkyl, hydroxy-C₁-C₆ alkyl, or amino-C₁-C₆ alkyl;
R¹² , R¹³, R¹⁴ and R¹⁵ are independently H or methyl;
R²¹ is H, methyl, C₁-C₆ alkyl, C₂-C₆ alkenyl, C₃-C₆ cycloalkyl or phenyl;
R²³ is H or C₁-C₆ alkyl;
R²⁴ is H, halogen, C₁-C₆ alkyl, C₂-C₆ alkenyl, -NHR²¹, hydroxy or C₁-C₆ alkoxy;
R²⁵ is C₁-C₆ alkyl;
wherein R²¹, R²⁴ and R²⁵ are optionally substituted one or more times with -OH,-CHO, or -CO₂H; and
wherein any two adjacent carbon atoms of R²¹, R²², R²⁴ and R²⁵, together with an oxygen atom, may form an epoxide;
R²², R²⁶, R²⁷, R²⁸ and R²⁹ are independently H, halogen, cyano, nitro, methyl, C₁-C₆ alkyl, C₂-C₆ alkenyl, -OR³⁰, -C(O)R³⁰, -OC(O)R³⁰, -C(O)OR³⁰, -S(O)R³⁰, -SO₂R³⁰, -SO₃R³⁰, -OSO₂R³⁰, -OSO₃R³⁰, -PO₂R³⁰R³¹, -OPO₂ R³⁰R³¹, -PO₃ R³⁰R³¹, -OPO₃ R³⁰R³¹, -N(R³⁰)R³¹, - C(O)N(R³⁰)R³¹, -C(O)NR³⁰NR³¹SO₂R³², -C(O)NR³⁰SO₂R³², -C(O)NR³⁰CN, -SO₂N(R³⁰)R³¹, -SO₂N(R³⁰)R³¹, -NR³²C(O)R³⁰, -NR³²C(O)OR³⁰ or -NR³²C(O)N(R³⁰)R³¹; or R²⁶ and R²⁷ are together -O-; or R²⁸ and R²⁷ together, or R²² and R²⁶ together, are independently -O-; or R²² and R²⁹ together are -O-; or R²⁸, together with the carbon to which it is bonded, is-C(=O)R³⁰, -CO₂R³⁰, -CH₂OR³⁰, -CH₂OC(O)R³⁰, -CH(OR³⁰)₂, -C(O)N(R³⁰R³¹), - C(=NR³¹)R³⁰, -C(=NOR³¹)R³⁰, or -C(=NNR³¹)R³⁰; provided that at least one pair of R²⁷ and R²⁸, R²⁶ and R²⁷, or R²² and R²⁶ do not form a ring; and
R³⁰, R³¹ and R³² are each independently -H or an optionally substituted aliphatic, cycloaliphatic, benzyl, or aryl, or -N(R³⁰)R³¹ together is an optionally substituted heterocyclic group, or -CH(OR³⁰)₂ together is a cyclic acetal group.

In one embodiment, R¹ is 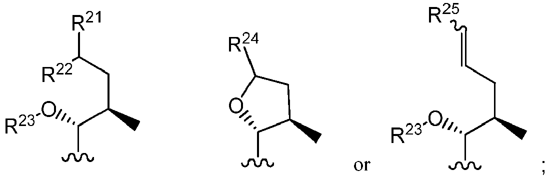
R² is C₁-C₆ alkyl, C₃-C₆ cycloalkyl, or hydroxy-C₁-C₆ alkyl;
R⁴, R⁵ and R¹¹ are independently C₁-C₆ alkyl or C₃-C₆ cycloalkyl;
R⁸ is C₁-C₆ alkyl, hydroxy-C₁-C₆ alkyl, or amino-C₁-C₆ alkyl;
R¹², R¹³, R¹⁴ and R¹⁵ are independently H or methyl;
R²¹ is H, methyl, C₁-C₆ alkyl, C₂-C₆ alkenyl, C₃-C₆ cycloalkyl or phenyl;
R²² is H, methyl, C₁-C₆ alkyl, C₂-C₆ alkenyl, hydroxy or C₁-C₆ alkoxy; or
R²³ is H or C₁-C₆ alkyl;
R²⁴ is H, halogen, C₁-C₆ alkyl, C₂-C₆ alkenyl, -NHR²¹ hydroxyl or C₁-C₆ alkoxy; and
R²⁵ is C₁-C₆ alkyl;
wherein R²¹, R²⁴ and R²⁵ are optionally substituted one or more times with -OH,-CHO, or -CO₂H; and
wherein any two adjacent carbon atoms of R²¹, R²², R²⁴ and R²⁵, together with an oxygen atom, may form an epoxide.

In another embodiment, R¹ is

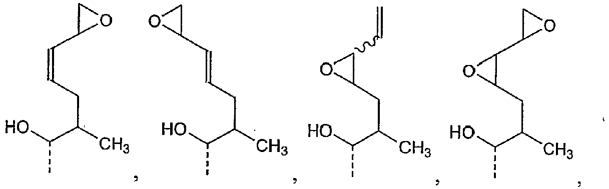

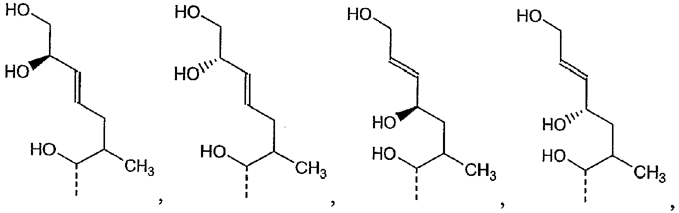

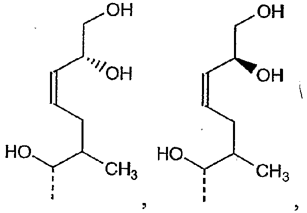

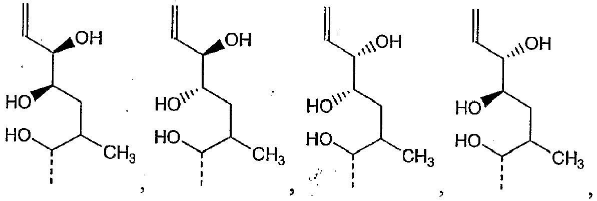

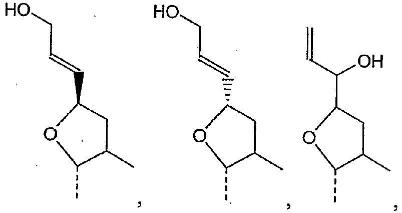

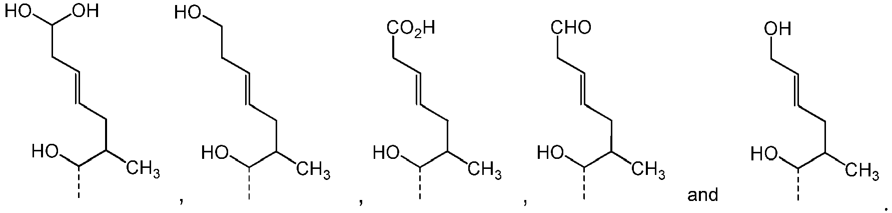

In still another embodiment, R¹ is

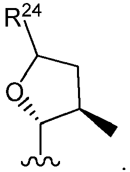

In another embodiment, R², R¹², R⁴, R⁵, R¹³, R⁸, R¹⁴, R¹⁵ and R¹¹ are C₁-C₆ alkyl.

In another embodiment,
R² is ethyl;
R⁴ is isobutyl;
R⁵ and R¹¹ are isopropyl; and
R⁸, R¹², R¹³, R¹⁴ and R¹⁵ are methyl.

In another aspect, the invention provides a cyclosporine H derivative of the Formula I and pharmaceutically acceptable salts and solvates thereof, wherein R¹ is CH(OH)CH(CH₃)CH₂CH₂R¹⁷R¹⁸; wherein
R¹⁷ is -CHCHCH₂CH₂-, -CHCHCH₂C(O)-, -(CH₂)₄- or -(CH₂)₃C(O)-; and
R¹⁸ is OH, O-C₁-C₆ alkyl, OC(O)-C₁-C₆ alkyl, NH₂, NH-C₁-C₆ alkyl or NHC(O)-C₁-C₆ alkyl;
R² is C₁-C₆ alkyl, C₃-C₆ cycloalkyl, or hydroxy-C₁-C₆ alkyl;
R⁴, R⁵ and R¹¹ are independently C₁-C₆ alkyl or C₃-C₆ cycloalkyl;
R⁸ is C₁-C₆ alkyl, hydroxy-C₁-C₆ alkyl, or amino-C₁-C₆ alkyl; and
R¹², R¹³, R¹⁴ and R¹⁵ are independently H or methyl.
In another embodiment, R¹ is CH(OH)CH(CH₃)CH₂CH₂R¹⁷R¹⁸; wherein
R¹⁷ is -CHCHCH₂CH₂-, -CHCHCH₂C(O)-, -(CH₂)₄- or -(CH₂)₃C(O)-; and
R¹⁸ is OH, OCH₃, O(Acetyl), NH₂, NH(CH₂CH₃) or NH(Acetyl);
R² is C₁-C₆ alkyl, C₃-C₆ cycloalkyl, or hydroxy-C₁-C₆ alkyl;
R⁴, R⁵ and R¹¹ are independently C₁-C₆ alkyl or C₃-C₆ cycloalkyl;
R⁸ is C₁-C₆ alkyl, hydroxy-C₁-C₆ alkyl, or amino-C₁-C₆ alkyl;
R¹², R¹³, R¹⁴ and R¹⁵ are independently H or methyl;

In a preferred embodiment, R¹ is

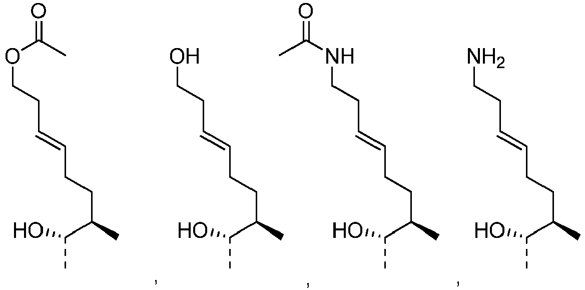

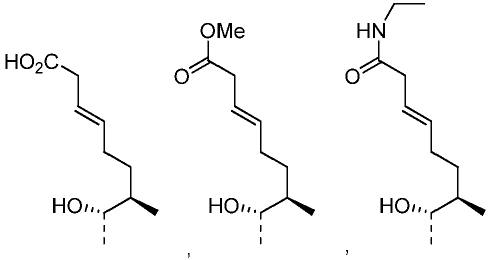

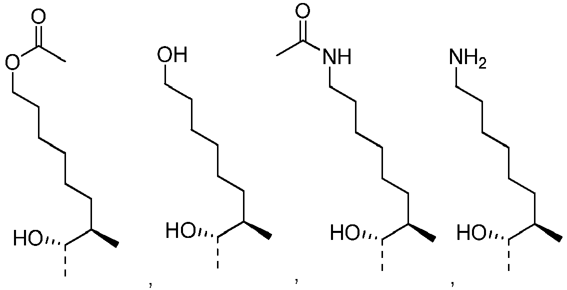

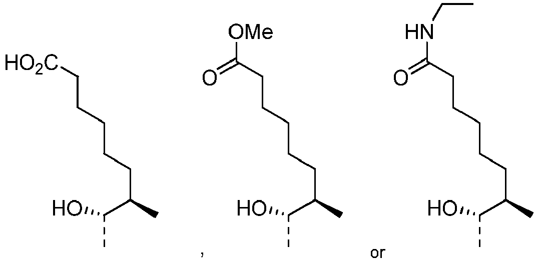

In another embodiment, R¹ is any of the substituted alkyl or alkylene groups described immediately above, and
R² is ethyl;
R⁴ is isobutyl;
R⁵ and R¹¹ are isopropyl; and
R⁸, R¹², R¹³, R¹⁴ and R¹⁵ are methyl.

It will be noted that the structures of some of the compounds of this invention include asymmetric carbon atoms. It is to be understood accordingly that the isomers arising from such asymmetry (*e.g*., all enantiomers and diastereomers) are included within the scope of this invention, unless indicated otherwise. Such isomers can be obtained in substantially pure form by classical separation techniques and by stereochemically controlled synthesis. Furthermore, the structures and other compounds and moieties discussed in this application also include all tautomers thereof. Compounds described herein can be obtained through art recognized synthesis strategies.

The description of the disclosure herein should be construed in congruity with the laws and principals of chemical bonding. For example, it may be necessary to remove a hydrogen atom in order accommodate a substitutent at any given location. Furthermore, it is to be understood that definitions of the variables *(i.e.,* "R groups"), as well as the bond locations of the generic formulae of the invention, will be consistent with the laws of chemical bonding known in the art. It is also to be understood that all of the compounds of the invention described above will further include bonds between adjacent atoms and/or hydrogens as required to satisfy the valence of each atom. That is, bonds and/or hydrogen atoms are added to provide the following number of total bonds to each of the following types of atoms: carbon: four bonds; nitrogen: three bonds; oxygen: two bonds; and sulfur: two-six bonds.

The compounds described herein can be obtained through art recognized synthesis strategies. See, for example, Wenger, Helvetica Chimica Acta 67 (Fasc. 2-Nr. 60): 502-525 and WO 2006/063470. Persons skilled in the art will recognize that the chemical reactions described herein may be readily adapted to prepare a number of other cyclosporin analogs of the invention, and alternative methods for preparing the compounds of this invention are deemed to be within the scope of this invention. For example, the synthesis of non-exemplified compounds according to the invention may be successfully performed by modifications apparent to those skilled in the art, *e.g.,* by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds of the invention.

Dihydrocyclosporin H is one preferred embodiment of this invention. As an example of method of production of dihydroCsH by hydrogenation of CsH, CsH is dissolved in methanol, and palladium on carbon (Pd/C) catalyst is added. This mixture is shaken on a Parr apparatus at room temperature under a hydrogen atmosphere until hydrogen uptake ceases. The mixture is filtered under a nitrogen atmosphere to remove catalyst, and the filtrate is concentrated *in vacuo* to remove methanol. The residue is dihydro-cyclosporin H may then be purified by chromatography or recrystallization as needed.

A "pharmaceutically acceptable salt," unless otherwise indicated, includes salts that retain the biological effectiveness of the free acids and bases of the specified compound and that are not biologically or otherwise undesirable. A compound of the invention may possess a sufficiently acidic, a sufficiently basic, or both functional groups, and accordingly react with any of a number of inorganic or organic bases, and inorganic and organic acids, to form a pharmaceutically acceptable salt. Examples of pharmaceutically acceptable salts include those salts prepared by reaction of the compounds of the present invention with a mineral or organic acid or an inorganic base, such salts including sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyn-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, .gamma.-hydroxybutyrates, glycollates, tartrates, methanesulfonates, propanesulfonates, naphthalene-1-sulfonates, naphthalene-2-sulfonates, and mandelates. Since a single compound of the present invention may include more than one acidic or basic moiety, the compounds of the present invention may include mono, di or tri-salts in a single compound.

If the inventive compound is a base, the desired pharmaceutically acceptable salt may be prepared by any suitable method available in the art, for example, treatment of the free base with an acidic compound, particularly an inorganic acid, such as hydrochloric acid, hydrobromic acid, sulfuric acid, nitric acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid such as glucuronic acid or galacturonic acid, an alpha hydroxy acid such as citric acid or tartaric acid, an amino acid such as aspartic acid or glutamic acid, an aromatic acid such as benzoic acid or cinnamic acid, a sulfonic acid such as p-toluenesulfonic acid or ethanesulfonic acid, or the like.

If the inventive compound is an acid, the desired pharmaceutically acceptable salt may be prepared by any suitable method, for example, treatment of the free acid with an inorganic or organic base. Examples of suitable inorganic salts include those formed with alkali and alkaline earth metals such as lithium, sodium, potassium, barium and calcium. Examples of suitable organic base salts include, for example, ammonium, dibenzylammonium, benzylammonium, 2-hydroxyethylammonium, bis(2-hydroxyethyl)ammonium, phenylethylbenzylamine, dibenzylethylenediamine, and the like salts. Other salts of acidic moieties may include, for example, those salts formed with procaine, quinine and N-methylglucosamine, plus salts formed with basic amino acids such as glycine, ornithine, histidine, phenylglycine, lysine and arginine.

As used herein, the term "alkyl" refers to a fully saturated branched or unbranched hydrocarbon moiety. Preferably the alkyl comprises 1 to 20 carbon atoms, more preferably 1 to 16 carbon atoms, 1 to 10 carbon atoms, 1 to 7 carbon atoms, or 1 to 4 carbon atoms. Representative examples of alkyl include, but are not limited to, methyl, ethyl, *n*-propyl, *iso-*propyl, *n*-butyl, *sec*-butyl, *iso*-butyl, *tert*-butyl, *n*-pentyl, isopentyl, neopentyl, *n*-hexyl, 3-methylhexyl, 2,2- dimethylpentyl, 2,3-dimethylpentyl, *n*-heptyl, *n*-octyl, *n*-nonyl, *n*-decyl and the like. Furthermore, the expression "Cₓ-C_{y}-alkyl", wherein x is 1-5 and y is 2-10 indicates a particular alkyl group (straight- or branched-chain) of a particular range of carbons. For example, the expression C₁-C₄-alkyl includes, but is not limited to, methyl, ethyl, propyl, butyl, isopropyl, tert-butyl and isobutyl.

The term "alkenyl," alone or in combination refers to a straight-chain, cyclic or branched hydrocarbon residue comprising at least one olefinic bond and the indicated number of carbon atoms. Preferred alkenyl groups have up to 8, preferably up to 6, particularly preferred up to 4 carbon atoms. Examples of alkenyl groups are ethenyl, 1-propenyl, 2-propenyl, isopropenyl, 1-butenyl, 2-butenyl, 3-butenyl, isobutenyl, 1-cyclohexenyl, 1-cyclopentenyl.

As used herein, the term "cycloalkyl" refers to saturated or unsaturated monocyclic, bicyclic or tricyclic hydrocarbon groups of 3-12 carbon atoms, preferably 3-9, or 3-7 carbon atoms. Exemplary monocyclic hydrocarbon groups include, but are not limited to, cyclopropyl, cyclobutyl, cyclopentyl, cyclopentenyl, cyclohexyl and cyclohexenyl and the like. Exemplary bicyclic hydrocarbon groups include bornyl, indyl, hexahydroindyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl and the like. Exemplary tricyclic hydrocarbon groups include adamantyl and the like.

The term "cycloalkenyl" refers to a partially unsaturated cyclic hydrocarbon group containing 1 to 3 rings and 4 to 8 carbons per ring. Exemplary groups include cyclobutenyl, cyclopentenyl, and cyclohexenyl. The term "cycloalkenyl" also includes bicyclic and tricyclic groups in which at least one of the rings is a partially unsaturated, carbon-containing ring and the second or third ring may be carbocyclic or heterocyclic, provided that the point of attachment is to the cycloalkenyl group.

"Alkoxy" refers to those alkyl groups, having from 1 to 10 carbon atoms, attached to the remainder of the molecule *via* an oxygen atom. Alkoxy groups with 1-8 carbon atoms are preferred. The alkyl portion of an alkoxy may be linear, cyclic, or branched, or a combination thereof. Examples of alkoxy groups include methoxy, ethoxy, isopropoxy, butoxy, cyclopentyloxy, and the like. An alkoxy group can also be represented by the following formula: -ORⁱ, where Rⁱ is the "alkyl portion" of an alkoxy group.

The term "aryl" includes aromatic monocyclic or multicyclic *e.g*., tricyclic, bicyclic, hydrocarbon ring systems consisting only of hydrogen and carbon and containing from six to nineteen carbon atoms, or six to ten carbon atoms, where the ring systems may be partially saturated. Aryl groups include, but are not limited to, groups such as phenyl, tolyl, xylyl, anthryl, naphthyl and phenanthryl. Aryl groups can also be fused or bridged with alicyclic or heterocyclic rings which are not aromatic so as to form a polycycle (*e.g*., tetralin).

The term "heteroaryl," as used herein, represents a stable monocyclic or bicyclic ring of up to 7 atoms in each ring, wherein at least one ring is aromatic and contains from 1 to 4 heteroatoms selected from the group consisting of O, N and S. Heteroaryl groups within the scope of this definition include but are not limited to: acridinyl, carbazolyl, cinnolinyl, quinoxalinyl, pyrrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, tetrahydroquinoline. As with the definition of heterocycle below, "heteroaryl" is also understood to include the N-oxide derivative of any nitrogen-containing heteroaryl. In cases where the heteroaryl substituent is bicyclic and one ring is non-aromatic or contains no heteroatoms, it is understood that attachment is via the aromatic ring or via the heteroatom containing ring, respectively.

The term "heterocycle" or "heterocyclyl" refers to a five-member to ten-member, fully saturated or partially unsaturated nonaromatic heterocylic groups containing at least one heteroatom such as O, S or N. The most frequent examples are piperidinyl, morpholinyl, piperazinyl, pyrrolidinyl or pirazinyl. Attachment of a heterocyclyl substituent can occur via a carbon atom or via a heteroatom.

Moreover, the alkyl, alkenyl, cycloalkyl, cycloalkenyl, alkoxy, aryl, heteroaryl, and heterocycle groups described above can be "unsubstituted" or "substituted." The term "substituted" is intended to describe moieties having substituents replacing a hydrogen on one or more atoms, *e.g.* C, O or N, of a molecule. Such substituents can independently include, for example, one or more of the following: straight or branched alkyl (preferably C₁-C₅), cycloalkyl (preferably C₃-C₈), alkoxy (preferably C₁-C₆), thioalkyl (preferably C₁-C₆), alkenyl (preferably C₂-C₆), alkynyl (preferably C₂-C₆), heterocyclic, carbocyclic, aryl (*e.g.,* phenyl), aryloxy (*e.g*., phenoxy), aralkyl (*e.g*., benzyl), aryloxyalkyl (*e.g*., phenyloxyalkyl), arylacetamidoyl, alkylaryl, heteroaralkyl, alkylcarbonyl and arylcarbonyl or other such acyl group, heteroarylcarbonyl, or heteroaryl group, (CR'R")₀₋₃NR'R" (*e.g*., -NH₂), (CR'R")₀₋₃CN (*e.g*., -CN), -NO₂, halogen (*e.g*., -F, -Cl, -Br, or -I), (CR'R")₀₋₃C(halogen)₃ (*e.g*., -CF₃), (CR'R")₀₋₃CH(halogen)₂, (CR'R")₀₋₃CH₂(halogen), (CR'R")₀₋₃CONR'R", (CR'R")₀₋₃(CNH)NR'R", (CR'R")₀₃S(O)₁₋₂NR'R", (CR'R")₀₋₃CHO, (CR'R")₀₋₃O(CR'R")₀₋₃H, (CR'R")₀₋₃S(O)₀₋₃R' (*e.g*., -SO₃H, -OSO₃H), (CR'R")₀₋₃O(CR'R")₀₋₃H (*e.g*., -CH₂OCH₃ and -OCH₃), (CR'R")₀₋₃S(CR'R")₀₋₃H *(e.g.,* -SH and -SCH₃), (CR'R")₀₋₃OH *(e.g.,* -OH), (CR'R")₀₋₃COR', (CR'R")₀₋₃(substituted or unsubstituted phenyl), (CR'R")₀₋₃(C₃-C₈ cycloalkyl), (CR'R")₀₋₃CO₂R' (*e.g*., -CO₂H), or (CR'R")₀₋₃OR' group, or the side chain of any naturally occurring amino acid; wherein R' and R" are each independently hydrogen, a C₁-C₅ alkyl, C₂-C₅ alkenyl, C₂-C₅ alkynyl, or aryl group.

The term "amine" or "amino" should be understood as being broadly applied to both a molecule, or a moiety or functional group, as generally understood in the art, and can be primary, secondary, or tertiary. The term "amine" or "amino" includes compounds where a nitrogen atom is covalently bonded to at least one carbon, hydrogen or heteroatom. The terms include, for example, but are not limited to, "alkyl amino," "arylamino," "diarylamino," "alkylarylamino," "alkylaminoaryl," "arylaminoalkyl," "alkaminoalkyl," "amide," "amido," and "aminocarbonyl." The term "alkyl amino" comprises groups and compounds wherein the nitrogen is bound to at least one additional alkyl group. The term "dialkyl amino" includes groups wherein the nitrogen atom is bound to at least two additional alkyl groups. The term "arylamino" and "diarylamino" include groups wherein the nitrogen is bound to at least one or two aryl groups, respectively. The term "alkylarylamino," "alkylaminoaryl" or "arylaminoalkyl" refers to an amino group which is bound to at least one alkyl group and at least one aryl group. The term "alkaminoalkyl" refers to an alkyl, alkenyl, or alkynyl group bound to a nitrogen atom which is also bound to an alkyl group.

The term "amide," "amido" or "aminocarbonyl" includes compounds or moieties which contain a nitrogen atom which is bound to the carbon of a carbonyl or a thiocarbonyl group. The term includes "alkaminocarbonyl" or "alkylaminocarbonyl" groups which include alkyl, alkenyl, aryl or alkynyl groups bound to an amino group bound to a carbonyl group. It includes arylaminocarbonyl and arylcarbonylamino groups which include aryl or heteroaryl moieties bound to an amino group which is bound to the carbon of a carbonyl or thiocarbonyl group. The terms "alkylaminocarbonyl," "alkenylaminocarbonyl," "alkynylaminocarbonyl," "arylaminocarbonyl," "alkylcarbonylamino," "alkenylcarbonylamino," "alkynylcarbonylamino," and "arylcarbonylamino" are included in term "amide." Amides also include urea groups (aminocarbonylamino) and carbamates (oxycarbonylamino).

In a particular embodiment of the invention, the term "amine" or "amino" refers to substituents of the formulas N(R')R" or C₁₋₆-N(R')R", wherein R and R are each, independently, selected from the group consisting of -H and -(C₁₋₄alkyl)₀₋₁G, wherein G is selected from the group consisting of -COOH, -H, -PO₃H, -SO₃H, -Br, -Cl, -F, -O-C₁₋₄alkyl, -S-C₁₋₄alkyl, aryl, -C(O)OC₁-C₆-alkyl, -C(O)C₁₋₄alkyl-COOH, -C(O)C₁-C₄-alkyl and-C(O)-aryl; or N(R')R" is pyrrolyl, tetrazolyl, pyrrolidinyl, pyrrolidinyl-2-one, dimethylpyrrolyl, imidazolyl and morpholino.

### Methods of Treatment

In the process of evaluating the safety of CsH as a potential therapeutic agent for neutrophilic disorders and neutrophil-mediated inflammatory processes, we observed a significant decrease of circulating eosinophils in animals to which CsH had been administered (see Table 1). This was a unique and unanticipated observation: although eosinophils are known to express the FPR, this receptor has never been implicated as having a role in mediating eosinophil maturation, release from bone marrow, systemically circulating levels, or localized recruitment.

A number of diseases are associated with eosinophil response and localization to specific tissues. In asthma, elevated levels of eosinophils are found both in circulation and localized in lung tissue. In HES and EGID disorders described below, elevated levels of eosinophils are observed. Moreover, inflammatory disorders of the skin (*e.g*. atopic dermatitis, psoriases, eczema) are associated with localized eosinophil infiltration. In each case, treatment with cyclosporines of this invention would decrease levels of circulating eosinophils and at localized sites associated with these respective disorder, as well as eosinophil activation at such sites; treatment with such compounds would ameliorate or alleviate such infiltration and associated symptoms.

In this invention, provided herein is the therapeutic use of FPR antagonists for the treatment of the eosinophil-associated diseases and disorders, including those enumerated above. In preferred embodiments, such antagonists would include CsH and its derivatives that act as potent and specific FPR antagonists (*e.g*. dihydro CsH, compounds of Formula I). Cyclosporines of this invention are administered at doses resulting in the reduction of circulating eosinophils, decrease in eosinophils at sites associated with diseases of increased eosinophil function or localized cell number, or a decrease in levels of eosinophil activation at such sites.

The term "eosinophil-associated disease" or "eosinophil-associated disorder" includes disorders and states (*e.g.,* a disease state) which are associated with aberrant levels or activation of eosinophils. Eosinophil-associated states include states resulting from an elevation in the expression of eosinophils. Examples of eosinophil-associated states include, but are not limited to, an immune disorder (*e.g*., eczema, dermatitis, or psoriasis) or an allergic disorder (*e.g*., asthma, hay fever, hypereosinophilic syndrome, or an eosinophil-associated gastrointestinal disorder), a gastrointestinal disorder (*e.g*., eosinophilic gastroenteritis, allergic colitis, eosinophilic esophagitis, inflammatory bowel disease or gastrointestinal reflux disease).

Other existing treatments of such eosinophilic disorders utilize systemic or localized steroids. Such treatments have limited efficacy and are accompanied by significant adverse side effects. FPR antagonists would also have an advantage over such steroidal treatment strategies, since agents specifically targeting this receptor would not manifest such side effects. FPR antagonists, including cyclosporines of this invention could be used in combination with steroidal treatments, and could allow treatment with lower doses of such therapeutic agents.

The cyclosporins of the invention are useful, for example, for the treatment of asthma of whatever type of genesis, including both intrinsic and extrinsic asthma. For example, they are useful for the treatment of atopic and non-atopic asthma, including allergic asthma, bronchitic asthma, exercise-induced asthma, occupational asthma, asthma induced following bacterial infection and other non-allergic asthmas. Treatment of asthma is also to be understood as embracing treatment of "wheezy-infant syndrome," that is treatment of subjects, *e.g*., of less than 4 to 5 years of age, exhibiting wheezing symptoms, in particular at night, and diagnosed or diagnosable as "wheezy infants," an established patient category of major medical concern and now more correctly identified as incipient or early-phase asthmatics. Cyclosporins of the invention are in additionally useful for the treatment of asthma in subjects whose asthmatic status is either steroid-dependent or steroid-resistant.

The term "asthma" has been used to describe a condition which is characterized by widespread fluctuations in the diameter or caliber of bronchial airways over short periods of time resulting in changes in lung function. The resulting increased resistance to air flow produces symptoms including breathlessness (dyspnea), chest constriction or "tightness" and wheeze. The term as used is not currently limited to a disorder or disease resulting from any specific cause or causes, rather it is characterized by its clinical manifestation. A true immunological mechanism may or may not be a factor in the etiology of an individual asthmatic condition. Further, characteristic wheezing may not be present in particularly severe attacks where transport of air is completely obstructed. Regardless of the cause, asthma in all sufferers is characterized by reversible hyperresponsiveness of tracheal bronchial smooth muscle resulting in its contraction and interference with normal respiration. The lungs of patients who die of asthma are usually pale pink, hyperinflated, and fail to collapse after their removal from the chest. Many of the airways throughout the bronchial tree are occluded by thick mucus plugs which are infiltrated with various types of leukocytes, including mast cells. The smooth muscle of the airways is hypertrophied. The bronchoconstriction or bronchospasm characterized by asthmatic attacks causes obstruction to air flow which necessitates a forced exhalation and maintenance of artificially elevated functional air reserve capacity to keep the airways open. The resultant lung hyperinflation places a significant stress on the cardiovascular system (particularly the right ventricle) which can lead to a consequent cardiovascular event.

Cyclosporins of the invention may be used for the treatment of eosinophil-related disorders of the airways (*e.g*., involving morbid eosinophilic infiltration of pulmonary tissues) including hypereosinophilia as it affects the airways and/or lungs as well as, for example, eosinophil-related disorders of the airways consequential or concomitant to Loffler's Syndrome, eosinophilic pneumonia, parasitic (in particular metazoan) infestation (including tropical eosinophilia), bronchopulmonary aspergillosis, polyarteritis nodosa (including Churg-Strauss Syndrome), eosinophilic granuloma and eosinophil-related disorders affecting the airways occasioned by drug reaction.

When used in relation to the treatment of diseases of the airways and lungs, in particular asthma, the term "treatment" is to be understood as embracing both symptomatic and prophylactic modes, that is the immediate treatment, for example, of acute inflammation (symptomatic treatment) as well as advance treatment to prevent, ameliorate or restrict long term symptomatology (prophylactic treatment). For example, in the case of asthma, the present invention includes symptomatic treatment to ameliorate acute inflammatory events as well as prophylactic treatment to inhibit on-going inflammatory status and to ameliorate future bronchial exacerbation associated therewith, including airway remodeling.

In chronic obstructive pulmonary disease (COPD) most of the airflow reduction is slowly progressive and irreversible because of damaged airway tissue. Increased neutrophils in bronchiolar lavage, as well as elevated levels of various markers of neutrophil activation are observed in COPD (reviewed by Barnes, 2000). These include neutrophil elastase (a destructive enzyme released by activated neutrophils), which is thought to play a key role in tissue damage in both COPD and emphysema.

Although cigarette smoking has been clearly identified as the major risk factor for COPD and emphysema, the molecular mechanisms by which smoking associated tissue damage manifest these diseases is not well understood. One major feature in the lungs of such patients is apoptosis; smoking has been shown to induce apoptosis of airway epithelial cells (reviewed in Hodge et al., 2004). It is known that apoptosis results in localized release and accumulation of cellular debris including, notably, mitochondrial materials.

Neutrophils and eosinophil infiltration predominate with progression of COPD, particularly during episodes of exacerbation and are thought to be involved in progressive tissue destruction associated with the disease (Papi et al., 2006). Moreover, mitochondrial formyl peptides can act as potent neutrophils activators, signaling through the formyl peptide receptor (Rabiet et el., 2005). Thus, in consideration of the neutrophil (and on occasion eosinophil) infiltration of lung tissue in patients suffering from COPD or emphysema, this invention describes a method of treatment of such patients by administration of a formyl peptide antagonist, which would attenuate activation mediated by formyl peptides in such tissues. Such antagonists could be used for long-term or prophylactic therapy, or in other cases, be used to treat disease exacerbations in such patients. The compounds of this invention include, but are not limited to CsH, dihydroCsH, and derivatives or analogs thereof (*e.g*. compounds of Formula 1).

Neutrophil activation can occur in response to a number of stimuli. IL-8, leukotrienes (*i.e.* LTB4), and C5a are known to be strong activators of neutrophils, acting as chemoattractants, motivating neutrophil extravasation. Another class of neutrophil activators is composed of formylated peptides: peptides composed in part by N-formyl-methionine (fMet). fMet is the first amino acid residue in the synthesis of proteins in prokaryotes and, consequently, is located at the N-terminus of the growing polypeptide. These proteins or degradation products thereof serve as potent activators of neutrophil recruitment and activation at sites of procaryotic (*i.e.* bacterial) infection.

N-formyl-methionine is also the initiator codon in mitochondria, which are evolutionary descendants of bacteria. Thus, proteins and peptides encoded and produced by mitochondria are also sources of fMet, and can stimulate neutrophils in a fashion analogous to those in procaryotes.

The receptor for N-formyl methionine containing peptides is known as the formyl peptide receptor (FPR). The FPR is a G protein coupled receptor (GPCR) expressed specifically on neutrophils, eosinophils, and basophils (i.e. granulocytes). Categorically, GPCRs have been the most successful target proteins for therapeutic intervention in disease processes: an estimated 30% of clinically prescribed drugs are GPCR agonists or antagonists (Stadel et al, Trends Pharmacol Sci. 18:430-437 (1997)). Although fMLF mediated chemoattraction is among the best known and characterized immunomodulatory signaling systems, little is known about the in vivo contribution of FPR signaling relative to other chemoattractants, including various components of the complement system and the many known chemokines, in various physiological scenarios of both health and disease. This is especially true of roles that FPR signaling might play in human diseases and disorders, including any etiologic roles in acute or chronic inflammation. Although inflammation is a critical event in localized immune response, serving to initiate and amplify signaling cascades that congregate and modulate cells and cellular activities necessary for clearing of an infection, loss of control of these signaling processes could potentially lead to any number of pathological inflammatory states or associated conditions. Thus, the potential of the FPR as a potential target for anti-inflammatory therapy is largely unexplored; FPR antagonists may be of considerable interest for use in treatment of a variety of inflammation-related disorders.

In addition to formylated peptide ligands, a number of FPR agonists and antagonists have been discovered. Antagonists include derivatized short Leu-Phe peptides, as well as cyclosporin H (CsH). CsH is a diastereomer of CsA that has .biological properties and activities that are clearly distinct from CsA: CsA inhibit T cell activation through inhibition of calcineurin mediated signaling. CsH has no effect on this signaling pathway, and has indeed been widely used as a negative control in studies of inhibition of immunosuppression by CsA. Moreover, CsA has no effect on FPR mediated signaling.

CF airway inflammation is characterized by unusually dense neutrophil infiltration and activation, which causes progressive airway damage through production and release of activated oxygen metabolites (*i.e.* hydrogen peroxide, oxygen radicals, ozone) and proteolytic enzymes, including neutrophil elastase (Fick, Chest 96: 158-164 (1989)). Moreover, excessive neutrophil response and consequent lung deterioration observed in CF patients may not be exclusively associated with *P. aeruginosa* infection itself. Signs of inflammation, including neutrophil and monocyte infiltration, ,and high levels of pro-inflammatory cytokines have been observed in children experiencing pulmonary inflammation in the absence of infection and in bronchoaveolar lavage (BAL) fluid of infants with CF prior to detectable *P. aeruginosa* infection (Khan et al., Am J Respir Crit Care Med 151:1075-82 (1995)).

Two altered states of *P. aeruginosa* growth are also thought to play a role in the disease course of CF patients: mucoid conversion and biofilm formation. Mucoid conversion occurs *in vivo* and is associated with establishment of chronic infection. Initial colonization of the CF lung by *P. aeruginosa* can often be eradicated by antibiotic therapy. However, in studies of sputum sample isolates at later times, a correlation has been observed between the appearance of colony morphology associated with conversion to mucoid growth and an inability to clear the infection, even with aggressive antibiotic treatment (Fredericksen et al., Pediatr. Pulmonol. 23:330-335 (1997)). The mucoid phenotype is characterized by overproduction of alginate, a capsule- like polysaccharide that is thought to affect bacterial adherence, manifest resistance to neutrophil attack, neutralize oxygen radicals, serve as a barrier to phagocytosis, and constitute a barrier that renders mucoid bacteria refractory to antibiotic therapy (Evans and Linker, J. Bacteriol 116:915-924, 1993; Govan and Deretic, Microbiol Rev 60:539-573 (1996)). The mucoid phenotype is often due to mutations that truncate the *mucA* gene product, and anti-sigma factor that normally negatively regulates the operon encoding alginate (Martin et al., Proc Natl Acad Sci USA 90:8377-8381 (1993)). mucA mutations are found in 84% of mucoid *P. aeruginosa* isolates (Boucher et al., Infect Immun 65:3838-46 (1997)), an observation that may account for the high incidence and persistence of mutator strains in CF patients (Oliver et al., Science 288:1251-53 (2000)).

Interestingly, one source of selection for mucoid conversion may be the presence of oxygen radicals produced by infiltrating neutrophils. Indeed, such radicals may play a causal role in the conversion of non-mucoid cells to the mucoid phenotype: transient exposure of *P. aeruginosa* to physiologically relevant concentrations of hydrogen peroxide *in vitro* leads to mucoid conversion (Mathee et al., Microbiology 145:1349-1357 (1999)). Thus, the exaggerated inflammatory immune response mounted by the CF patient in the infected lung may play an additional role in the disease process by influencing the emergence of intractable forms of *P. aeruginosa.* This, in turn, suggests that selective attenuation of neutrophil response in such patients might prevent or delay mucoid conversion, thereby perpetuating the efficacy of antibiotic therapies and delay or prevent conversion of non-mucoid *P. aeruginosa* to the more virulent mucoid form.

One desirable ultimate goal of an effective therapy for inflammation in the CF lung would be the attenuation of neutrophil elastase release and oxide radical production without profoundly diminishing other neutrophil functions or the responsiveness of other components of the immune response, thereby maintaining the ability to effectively combat the bacterial infection while minimizing inflammation-related tissue damage of the lung. As mentioned above, neutrophil recruitment and activation is mediated by several small signaling molecules that form extracellular gradients. However, formylated peptides differ from less potent signaling molecules in that they elaborate a robust and complete neutrophil response at extremely low concentrations, In contrast, other effectors, such as C5a, leukotrienes, and IL-8, potentially stimulate chemotaxis and antigenic body engulfment, but require considerably higher concentrations to effectively induce degranulation and superoxide production (Klinker et al., Biochem Pharmacol 48:1857-1864 (1994)).

As described above, *P. aeruginosa* may be thought of as an unfortunate (and pathogenic) component of the flora of the CF lung. As such, another site rich in bacterial flora is the digestive system, where residence of various bacteria and microorganisms is always found, and indeed, associated with good digestive health and well-being. Such indigenous flora aid in digestion, as well as fend off colonization or growth of non-beneficial or harmful microorganisms.

As a site of high levels of microorganisms, most if not all of which although beneficial, would normally provoke immune response, the gut must exercise a number of mechanisms to control immune response to such indigenous flora. Moreover, digestive products from the intestines and colon must be taken up systemically. Thus, the mucosal lining of the digestive system serves as an important semipermeable barrier, allowing transport of nutrients and digestive products, but excluding cells that mediate immune response by serving as a physical barrier separating them from microorganisms in the digestive tract.

The physical integrity of the mucosal lining of the digestive tract is therefore, very important; injury or insult might lead to pathological conditions. It has been suggested that inflammation in various forms of colitis, Crohn's disease or inflammatory bowel disease (IBD) is due to increased permeability of the inner lining of the colon or intestine, which may allow bacteria to locally penetrate the tissues and cause an immune reaction that leads prolonged inflammation. Alternatively, immunostimulatory molecules produced by these organisms may be allowed to permeate nearby tissue, thereby provoking immune response. As neutrophils accumulate and become activated and other aspects of immune response also become engaged, the resulting tissue damage becomes a self-perpetuating lesion in which tissue has no opportunity to heal and resolve the inflammatory immune response.

Because formyl peptides are present at high concentrations at sites of bacterial colonization, it highly likely that these molecules, produced by the indigenous microbial flora of the digestive tract, play a role in recruitment of neutrophils to sites of insult and lesions associates with colitis, Crohn's disease, and IBD. This is substantiated by the observation that neutrophil infiltration is a hallmark of these disorders. By treating patients with an FPR antagonist, this cycle of neutrophil recruitment and activation, inflammation, and tissue damage may be resolved, such that lesions heal and the necessary barrier between intestinal flora and the immune system is reconstituted.

Another class of potent activators of the FPR is the peptides containing the initiator methionine of proteins encoded and produced by mitochondria. In areas of localized tissue damage, especially those associated with apoptosis, such mitochondrial products are released and may provoke a granulocyte *(i.e.* neutrophil)- mediated immune response through FPR signaling. One example of such injury and response is found in ischemic tissue, where apoptosis and localized neutrophil infiltration has been observed. Examples of such sites of ischemic injury are tissue reperfusion injury in transplanted organs, myocardial infarction or stroke. By pretreating patients donating organs, perfusing donated organs, pretreating and/or treating organ recipients with FPR antagonists, the inflammatory sequellae of tissue injury and apoptosis associated with reperfusion may be attenuated or prevented due to inhibition of neutrophil infiltration or activation, minimizing or preventing tissue injury. Similarly, treatment of patients suffering stroke or heart attack (*i.e.* myocardial infarction) with FPR antagonists after an ischemic event would minimize damage to those tissues.

Provided herein is the use of formyl peptide receptor antagonists in the therapy of diseases or disorders involving inappropriate, exaggerated, or prolonged neutrophil response (i.e. recruitment and/or activation). Cyclosporin H (CsH) is a known potent and specific antagonist of the formyl peptide receptor, and therefore can be used therapeutically as an antagonist for the indications described herein. Similarly, derivatives and analogs of CsH with FPR antagonistic activity would also have therapeutic use. Moreover, CsH like CsA, CsH contains a MeBmt side chain. In contrast to CsA, in which this side chin is quite intolerant of chemical modification to maintain biological activity (modifications of this side chain in CsA have focused almost exclusively on generating biologically inactive prodrugs that are then metabolized into biologically active molecules), we have observed that the MeBmt moiety of CsH can be chemically altered and maintain biological function (i.e. retain FPR inhibitory properties). For example, reducing the double bond in the MeBmt side chain of CsA results in dramatic loss of activity in T cell activation assays. Crystal structure studies have shown that this side chain has four points of specific interaction with the cyclophilin component of the cyclophilin- CsA- calcineurin complex (Huai et al., Proc Natl Acad Sci USA 99:12037-12042 (2002)). Reduction of this same bond in CsH has no effect on the apparent IC50 of CsH in binding assays of formyl peptide to the human formyl peptide receptor. Thus the cyclosporins of this invention include dihydroCsH, as well as compounds of Formula I that maintain FPR inhibitory activity.

Based on our observation of the tolerability of the MeBmt side chain to chemical modification in maintaining the FPR binding activity of CsH, we propose chemical modification of this side chain as a means of beneficially altering the ADME (absorption, distribution, metabolism, and excretion), solubility, bioavailability, or pharmacokinetic (PK) characteristics of CsH in treated patients. Such alterations would also offer a strategy of attenuating or modulating drug-drug interactions between CsH and other concomitantly administered therapies (*i.e.* drug-drug interactions, altered metabolism, absorption or excretion of other co-administered drugs). Moreover, the MeBMT side chain is well documented as a site of cyclosporin metabolism; chemical modification or alteration of this moiety would be expected to have an effect on metabolism of CsH-based compounds.

CsA is also known to be a substrate of the p-glycoprotein transporter, and this property is thought to be associated with drug-drug interactions as well as both its absorption and excretion characteristics of those of co-administered drugs. As disclosed here, the lack of activity of CsH is a desirable property that distinguishes this class of compounds from CsA and its biologically active derivatives or analogs (*i.e*., those that retain activity in T cell activation or cyclophilin- calcineurin pathway signaling assays).

The invention is also directed to pharmaceutically active metabolites and pharmaceutically acceptable salts of compounds of CsH, dihydroCsH, or Formula I. The invention also relates to pharmaceutical compositions comprising a therapeutically effective amount of a compound of CsH, dihydroCsH, or Formula I or a metabolite, solvate, resolved enantiomer, diastereomer, racemic mixture or pharmaceutically acceptable salt thereof.

The inventive compounds may be used advantageously in combination with other known therapeutic agents. Accordingly, this invention also relates to pharmaceutical compositions comprising a therapeutically effective amount of a compound of CsH, dihydroCsH, or Formula I or a metabolite, solvate, resolved enantiomer, diastereomer, racemic mixture or pharmaceutically acceptable salt thereof, in combination with a second therapeutic agent.

In a further aspect the present invention provides a method of using the compounds of this invention as a medicament to treat respiratory diseases, disorders, and conditions. For example, this invention provides a method for treatment of an immunoregulatory or respiratory disorder in a mammal comprising administrating to said mammal one or more compounds of CsH, dihydroCsH, or Formula I, or a metabolite, solvate, resolved enantiomer, diastereomer, racemic mixture or pharmaceutically acceptable salt thereof, in an amount effective to treat said disorder. Other aspects similarly include methods for treating diseases of the digestive tract or skin.

This invention further provides kits comprising one or more compounds of CsH, dihydroCsH, or Formula I, or a metabolite, solvate, resolved enantiomer, diastereomer, racemic mixture or pharmaceutically acceptable salt thereof. The kit may further comprise a second compound or formulation comprising a second pharmaceutical agent for treating an immunoregulatory or respiratory disease, disorder, or condition.

A "pharmaceutically active metabolite" is a pharmacologically active product produced through metabolism in the body of a specified compound or salt thereof. Metabolites of a compound may be identified using routine techniques known in the art and their activities determined using tests such as those described herein. Active metabolites of a compound may be identified using routine techniques known in the art.

The cyclosporins of the present invention are useful for the treatment of diseases or conditions responsive to or requiring anti-inflammatory or related therapy, *e.g.* for topical administration for the treatment of such diseases or conditions of the eye, nasal passages, buccal cavity, colon, skin, intestinal tract, airway, lung, ear, anus or vagina. Cyclosporins of the invention are useful, for example, for the treatment of diseases and conditions of the airways or lung, in particular inflammatory or obstructive airways disease. They are especially useful for the treatment of diseases or conditions of the airways or lung associated with or characterized by inflammatory cell infiltration or other inflammatory event accompanied by the accumulation of inflammatory cells, *e.g.,* eosinophils.

The term "treating" as used herein, unless otherwise indicated, means reversing, alleviating, inhibiting the progress of, or preventing the disorder or condition to which such term applies, or one or more symptoms of such disorder or condition. The term "treatment," as used herein, unless otherwise indicated, refers to the act of treating as "treating" is defined immediately above. "Treating" is intended to mean at least the mitigation of a disease condition in a mammal, such as a human, particularly when the mammal is found to be predisposed to having the disease condition but has not yet been diagnosed as having it and includes, but is not limited to, modulating and/or inhibiting the disease condition; and/or alleviating the disease condition or symptoms thereof.

### Pharmaceutical Compositions

The language "effective amount" of the compound is that amount necessary or sufficient to treat or prevent an eosinophil-associated or neutrophil-associated disease, *e.g.* prevent the various morphological and somatic symptoms of an eosinophil-associated or neutrophil-associated disease, and/or a disease or condition described herein. In an example, an effective amount of the compound of the invention is the amount sufficient to treat an eosinophil-associated or neutrophil-associated disease in a subject. The effective amount can vary depending on such factors as the size and weight of the subject, the type of illness, or the particular compound of the invention. For example, the choice of the compound of the invention can affect what constitutes an "effective amount." One of ordinary skill in the art would be able to study the factors contained herein and make the determination regarding the effective amount of the compounds of the invention without undue experimentation.

The regimen of administration can affect what constitutes an effective amount. The compound of the invention can be administered to the subject either prior to or after the onset of an eosinophil-associated or neutrophil-associated disease. Further, several divided dosages, as well as staggered dosages, can be administered daily or sequentially, or the dose can be continuously infused, or can be a bolus injection. Further, the dosages of the compound(s) of the invention can be proportionally increased or decreased as indicated by the exigencies of the therapeutic or prophylactic situation.

Cyclosporins of the invention may be administered by routes including, but not limited to, the pulmonary route (inhalation), nasal administration, rectal administration (*e.g*., suppository or enema form), dermally (topically to the skin), orally or ophthalmically.

For example, CsH, dihydroCsH, or compounds of Formula I can be administered topically within the airways, *e.g*. by the pulmonary route, by inhalation. Compounds of the inventions can also be administered dermally, i.e. topically to the skin, for example for the treatment of cutaneous diseases mediated by immune mechanisms, *e.g.,* psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lumpus erythematosus, pemphisus, epidermolysis bullosa acquisita, and other inflammatory or allergic conditions of the skin. Optionally, the cyclosporins of the invention are co-administered together with anti-inflammatory, immunosuppressive, or other pharmacologically active agents, *e.g.,* corticosteroids, antihistamines, antibiotics, antifungals, etc.

In order to use a compound of the invention, it is normally formulated in accordance with standard pharmaceutical practice as a pharmaceutical composition. According to this aspect of the invention there is provided a pharmaceutical composition that comprises CsH, dihydroCsH, or a compound of Formula I (or a pharmaceutically acceptable salt thereof), in association with a pharmaceutically acceptable diluent or carrier.

To prepare the pharmaceutical compositions according to this invention, a therapeutically or prophylactically effective amount of CsH, dihydroCsH, or a compound of Formula I or pharmaceutically acceptable salt, solvate, or metabolite thereof (alone or together with an additional therapeutic agent as disclosed herein) the compound is intimately admixed, for example, with a pharmaceutically acceptable carrier according to conventional pharmaceutical compounding techniques to produce a dose. A carrier may take a wide variety of forms depending on the form of preparation desired for administration, *e.g*., oral or parenteral. Examples of suitable carriers include, but are not limited to, any and all solvents, dispersion media, adjuvants, coatings, antibacterial and antifungal agents, isotonic and absorption delaying agents, sweeteners, stabilizers (to promote long term storage), emulsifiers, binding agents, thickening agents, salts, preservatives, solvents, dispersion media, coatings, antibacterial and antifungal agents, isotonic and absorption delaying agents, flavoring agents, and miscellaneous materials such as buffers and absorbents that may be needed in order to prepare a particular therapeutic composition. The use of such media and agents with pharmaceutically active substances is well known in the art. Except insofar as any conventional media or agent is incompatible with a compound of this invention, its use in the therapeutic compositions and preparations is contemplated. Supplementary active ingredients can also be incorporated into the compositions and preparations as described herein.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, or intramuscular dosing or as a suppository for rectal dosing). For example, compositions intended for oral use may contain, for example, one or more coloring, sweetening, flavoring and/or preservative agents.

Suitable pharmaceutically-acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), coloring agents, flavoring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavoring and coloring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavoring and preservative agents.

Syrups and elixirs may be formulated with sweetening agents such as glycerol, propylene glycol, sorbitol, aspartame or sucrose, and may also contain a demulcent, preservative, flavoring and/or coloring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Suppository formulations may be prepared by mixing the active ingredient with a suitable non-irritating excipient that is solid at ordinary temperatures but liquid at the rectal temperature and will therefore melt in the rectum to release the drug. Suitable excipients include, for example, cocoa butter and polyethylene glycols.

Topical formulations, such as creams, ointments, gels and aqueous or oily solutions or suspensions, may generally be obtained by formulating an active ingredient with a conventional, topically acceptable, vehicle or diluent using conventional procedures well known in the art.

Compositions for administration by insufflation may be in the form of a finely divided powder containing particles of average diameter of, for example, 30 .mu.m or much less, the powder itself comprising either active ingredient alone or diluted with one or more physiologically acceptable carriers such as lactose. The powder for insufflation is then conveniently retained in a capsule containing, for example, 1 to 50 mg of active ingredient for use with a turbo-inhaler device, such as is used for insufflation of the known agent sodium cromoglycate.

Compositions for administration by inhalation may be in the form of a conventional pressurized aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

Use of controlled-release oral dosage forms that comprise a tablet or capsule containing a plurality of particles of a cyclosporin of this invention dispersed in a swellable/erodible polymer may be used. Further, controlled release oral dosage forms of the cyclosporins of the invention may be used for continuous, sustained administration to the upper gastrointestinal tract of a patient. The majority of the dose of cyclosporins of the invention may be delivered, on an extended release basis, to the stomach, duodenum, and upper regions of the small intestine, with delivery of the drug to the lower gastrointestinal tract and colon substantially restricted. A variety of technologies, including hydrophilic, water-swellable, crosslinked, polymers that maintain physical integrity over the dosage lifetime but thereafter rapidly dissolve may be utilized for delivery of the cyclosporins of the invention.

For further information on formulations, see Chapter 25.2 in Volume 5 of Comprehensive Medicinal Chemistry (Corwin Hansch; Chairman of Editorial Board), Pergamon Press 1990.

It will be understood that the specific dosage level and frequency of dosage therapeutic or prophylactic purposes for any particular subject may be varied and will depend upon a variety of factors including the activity of the specific compound (i.e. CsH, dihydroCsH, or compounds of Formula I), the species, age, body weight, general health, sex and diet of the subject, the rate, mode and time of administration, rate of excretion, drug combination, severity of the particular condition, and the discretion of the prescribing physician, but can nevertheless be routinely determined by one skilled in the art. In one embodiment, an effective dosage is in the range of about 0.001 to about 100 mg per kg body weight per day, for example, about 0.5 to about 35 mg/kg/day, in single or divided doses. For a 70 kg human, this would amount to about 0.0035 to 2.5 g/day, such as about 0.05 to about 2.5 g/day. In some instances, dosage levels below the lower limit of the aforesaid range may be more than adequate, while in other cases still larger doses may be employed without causing any harmful side effect, provided that such larger doses are first divided into several small doses for administration throughout the day. For further information on routes of administration and dosage regimes, see Chapter 25.3 in Volume 5 of Comprehensive Medicinal Chemistry (Corwin Hansch; Chairman of Editorial Board), Pergamon Press 1990.

This invention further provides CsH, dihydroCsH, or a compound of Formula I or a metabolite, solvate, resolved enantiomer, diastereomer, racemic mixture or pharmaceutically acceptable salt thereof for use as a medicament in the treatment of the diseases or conditions described above in a warm-blooded animal, such as a mammal, for example, a human, suffering from such disease or condition. Also provided is the use of CsH, dihydroCsH, or a compound of Formula I or a metabolite, solvate, resolved enantiomer, diastereomer, racemic mixture or pharmaceutically acceptable salt thereof in the preparation of a medicament for the treatment of the diseases and conditions described above in a warm-blooded animal, such as a mammal, for example a human, suffering from such disorder.

The active compounds may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The active compounds may also be coupled with soluble polymers as targetable drug carriers. Such polymers can include polyvinylpyrrolidone, pyran copolymer, polyhydroxypropylmethacrylamide phenyl, polyhydroxyethylaspartamide-phenol, or polyethyleneoxide-polylysine substituted with palmitoylresidues. Furthermore, the active compounds may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross-linked or amphipathic block copolymers of hydrogels.

### Kits

In another embodiment of the invention, an article of manufacture, or "kit," containing materials useful for the treatment of the disorders described above is provided. In one embodiment, the kit comprises a container comprising a compound of this invention. In one embodiment, the invention provides a kit for treating immunoregulatory or respiratory diseases, disorders, or conditions involving neutrophil activation. Suitable containers include, for example, bottles, vials, syringes, blister pack, etc. The container may be formed from a variety of materials such as glass or plastic. The container holds a compound of this invention or a pharmaceutical formulation thereof, in an amount effective for treating the condition, and may have a sterile access port (for example, the container may be an intravenous solution bag or a vial having a stopper pierceable by a hypodermic injection needle). The kit may further comprise a label or package insert on or associated with the container. The label or package insert indicates that the composition is used for treating the condition of choice. Alternatively, or additionally, the kit may further comprise a second container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The kit may further comprise directions for the administration of the compound of this invention and, if present, the second pharmaceutical formulation for treating or preventing an immunoregulatory or respiratory disease, disorder or condition. For example, if the kit comprises a first composition comprising a compound of this invention and a second pharmaceutical formulation, the kit may further comprise directions for the simultaneous, sequential or separate administration of the first and second pharmaceutical compositions to a patient in need thereof.

In another embodiment, the kits are suitable for the delivery of solid oral forms of a compound of this invention, such as tablets or capsules. Such a kit includes, for example, a number of unit dosages. Such kits can include a card having the dosages oriented in the order of their intended use. An example of such a kit is a "blister pack". Blister packs are well known in the packaging industry and are widely used for packaging pharmaceutical unit dosage forms. If desired, a memory aid can be provided, for example in the form of numbers, letters, or other markings or with a calendar insert, designating the days in the treatment schedule in which the dosages can be administered.

According to one embodiment, the kit may comprise (a) a first container with a compound of this invention contained therein; and optionally (b) a second pharmaceutical formulation, wherein the second pharmaceutical formulation comprises a second compound for treatment of the immunoregulatory or respiratory disease, disorder, or condition associated with neutrophil activation. Alternatively, or additionally, the kit may further comprise a third container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

In certain other embodiments wherein the kit comprises a pharmaceutical formulation of a compound of this invention and a second formulation comprising a second therapeutic agent, the kit may comprise a separate container for containing the separate formulations, such as a divided bottle or a divided foil packet; however, the separate compositions may also be contained within a single, undivided container. Typically, the kit comprises directions for the administration of the separate components. The kit form is particularly advantageous when the separate components are administered in different dosage forms *(e.g.,* oral and parenteral), are administered at different dosage intervals, or when titration of the individual components of the combination is desired by the prescribing physician.

**Table 1. Summary of Hematology Data**

| A Multiple Oral Gavage Toxicity Study of Cyclosporin H in Sprague-Dawley Rats with Toxicokinetics | | | | | | | | | | | | | |
|---|---|---|---|---|---|---|---|---|---|---|---|---|---|
| **Group** | **Sex** | | **LYM %** Percentage | **MONO** 10^3/uL | **MON%** Percentage | **BASO** 10^9/L | **BAS%** Percentage | **EOS** 10^3/uL | **EOS %** Percentage | **PLT** 10^3/uL | **MPV** fL | **RETIC** 10^9/L | **%RETIC** Percentage |
| Vehicle | M | Mean | 81.85 | 0.233 | 2.13 | 0.043 | 0.48 | 0.270 | 3.25 | 1147.0 | 8.05 | 297.10 | 3.630 |
| | | S.D. N | 6.48 | 0.177 | 1.00 | 0.013 | 0.17 | 0.182 | 3.40 | 254.7 | 0.66 | 50.28 | 0.539 |
| | | | 4 | 4 | 4 | 4 | 4 | 4 | 4 | 4 | 4 | 4 | 4 |
| 30mg/kg | M | Mean | 80.50 | 0.333 | 3.05 | 0.028 | 0.25 | | 0.58 | 1267.5 | 7.30 | 407.63* | 5.848** |
| | | | | | | | | 0.060* | | | | | |
| | | S.D. N | 4.07 | 0.182 | 0.54 | 0.015 | 0.06 | 0.061 | 0.39 | 371.2 | 0.85 | 21.61 | 0.406 |
| | | | 4 | 4 | 4 | 4 | 4 | 4 | 4 | 4 | 4 | 4 | 4 |
| 100mg/kg | M | Mean | 81.60 | 0.490 | 4.68* | 0.038 | 0.30 | | 0.45 | 1511.3 | 9.65* | 427.15* | 6.035** |
| | | | | | | | | 0.050* | | | | | |
| | | S.D. N | 2.94 | 0.274 | 1.36 | 0.028 | 0.14 | 0.039 | 0.21 | 227.8 | 0.41 | 70.62 | 0.947 |
| | | | 4 | 4 | 4 | 4 | 4 | 4 | 4 | 4 | 4 | 4 | 4 |
| Vehicle | F | Mean | 71.35 | 0.268 | 2.83 | 0.040 | 0.45 | 0.310 | 6.53 | 1070.8 | 8.30 | 232.68 | 3.023 |
| | | S.D. N | 15.72 | 0.211 | 1.07 | 0.032 | 0.13 | 0.263 | 5.89 | 87.6 | 0.22 | 46.08 | 0.544 |
| | | | 4 | 4 | 4 | 4 | 4 | 4 | 4 | 4 | 4 | 4 | 4 |
| 30mg/kg | F | Mean | 81.20 | 0.243 | 3.43 | 0.018 | 0.25* | 0.080 | 1.23 | | 7.88 | 391.65** | 5.650** |
| | | | | | | | | | | 1436.0* | | | |
| | | S.D. N | 2.85 | 0.136 | 0.81 | 0.010 | 0.06 | 0.014 | 0.34 | 135.8 | 1.18 | 67.49 | 0.992 |
| | | | 4 | 4 | 4 | 4 | 4 | 4 | 4 | 4 | 4 | 4 | 4 |
| 100mg/kg | F | Mean | 61.40 | 0.340 | 6.40 | 0.020 | 0.38 | 0.038 | 0.75 | | 8.33 | 345.65* | 5.188** |
| | | | | | | | | | | 1434.5* | | | |
| | | S.D. N | 36.98 | 0.281 | 4.45 | 0.008 | 0.10 | 0.033 | 0.44 | 188.5 | 0.48 | 22.37 | 0.378 |
| | | | 4 | 4 | 4 | 4 | 4 | 4 | 4 | 4 | 4 | 4 | 4 |

### Prophetic Synthesis Examples

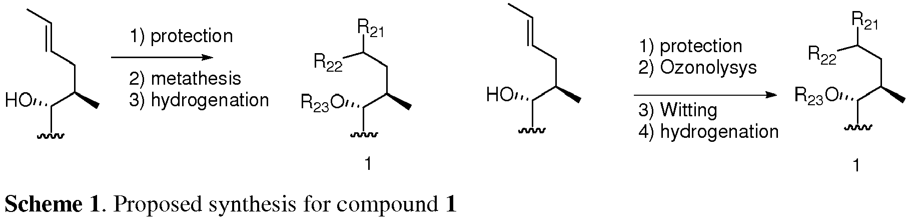

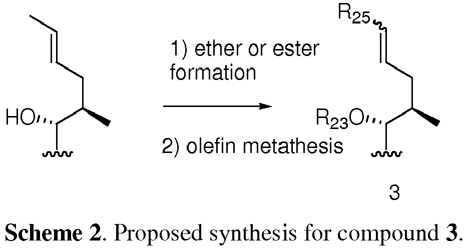

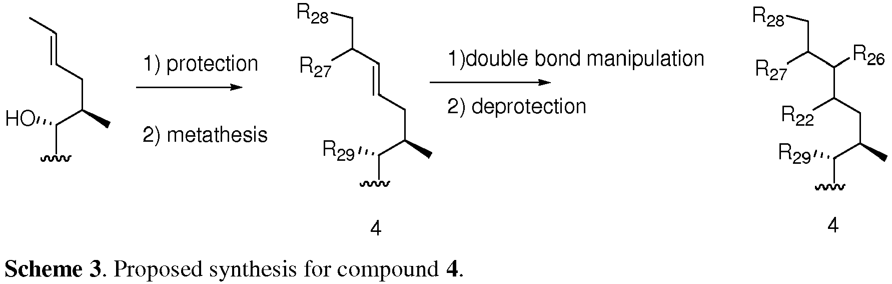

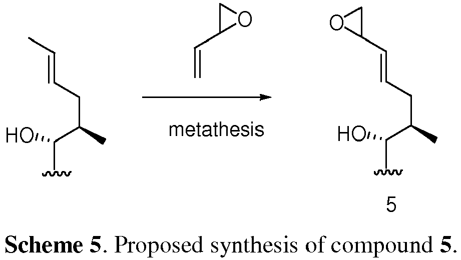

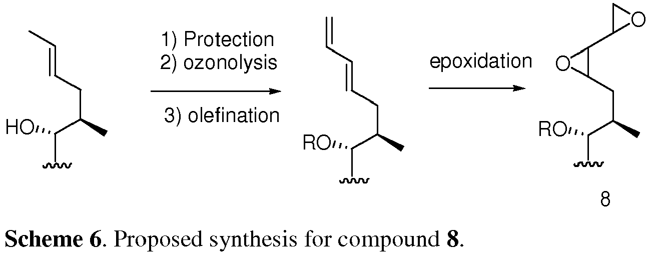

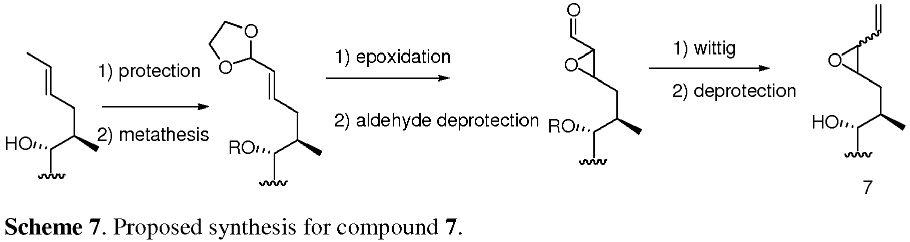

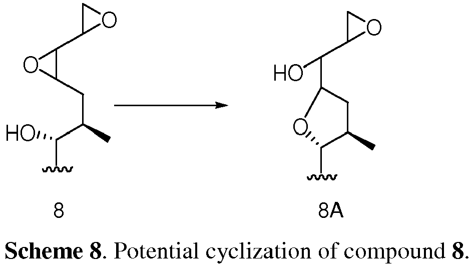

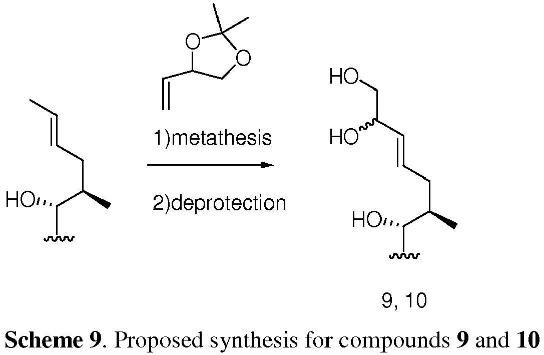

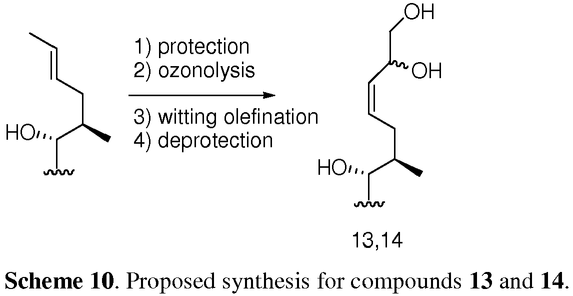

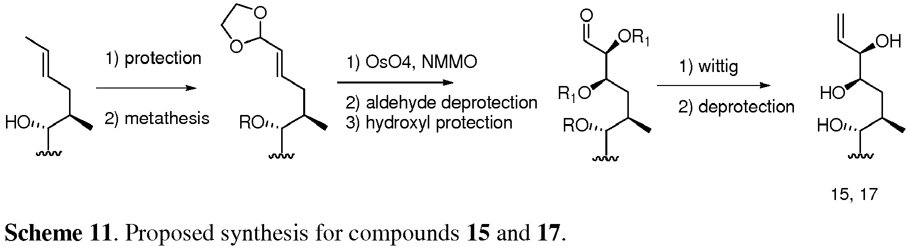

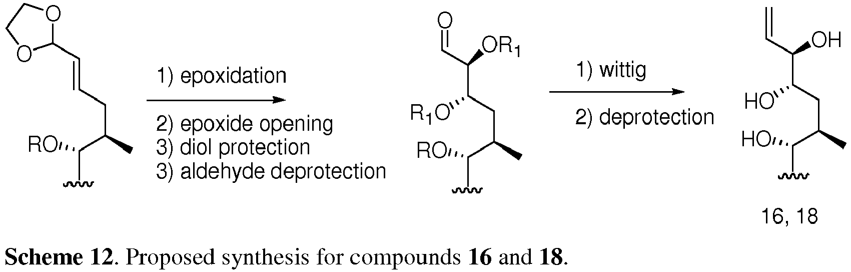

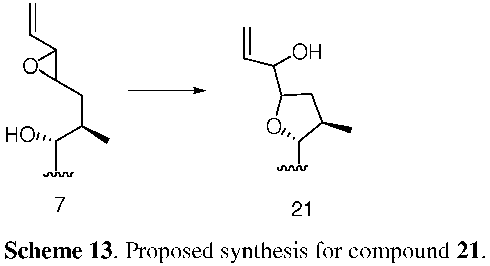

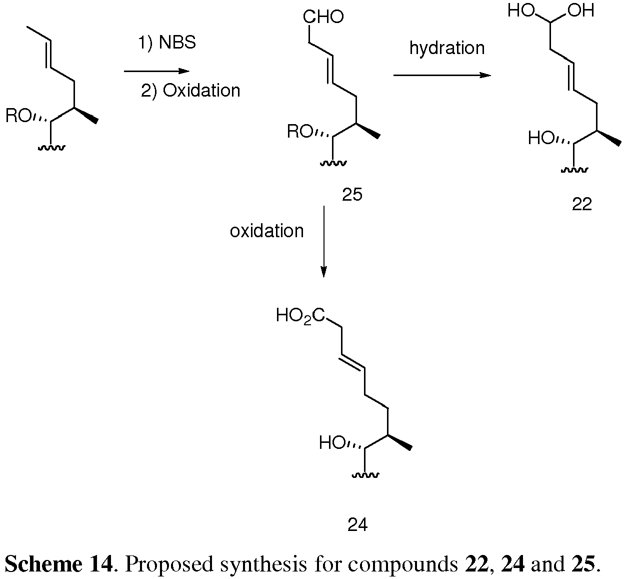

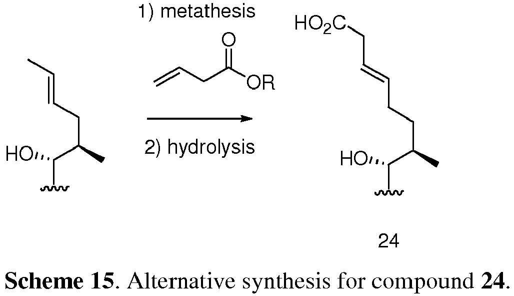

### References

Hogan, S.P. and M.E. Rothenberg. Eosinophil Function in Eosinophil- associated Gastrointestinal Disorders. Curr. Allergy and Asthma Reports 6:65-71 (2006).
Wenzel-Seifert, K., L. Grunbaum, and R. Seifert. Differential Inhibition of Human Neutrophil Activation by Cyclosporine A, D, and H. Journal of Immunology 147:1940-1946 (1991).
Wenzel-Seifert, K. and R. Seifert. Cyclosporin H is a Potent and Selective Formyl Peptide Receptor Antagonist. Journal of Immunology 150:4591-4599 (1993).
Novak, N., T. Bieber, and D.Y.M. Leung. Immune Mechanisms Leading to Atopic Dermatitis. J. Allergy Clin.Immunol. 112: S128-S139 (2003).
Simon, D., L.R. Braathen, and H.-U. Simon. Eosinophils and Atopic Dermatitis. Allergy 59: 561-570 (2004).

## Claims

1. A cyclosporine H derivative of formula I: 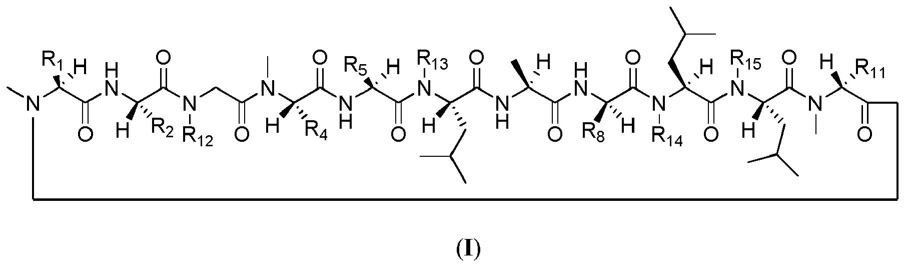 and pharmaceutically acceptable salts and solvates thereof, wherein
R¹ is CH(OH)CH(CH₃)CH₂CH₂R¹⁷R¹⁸, 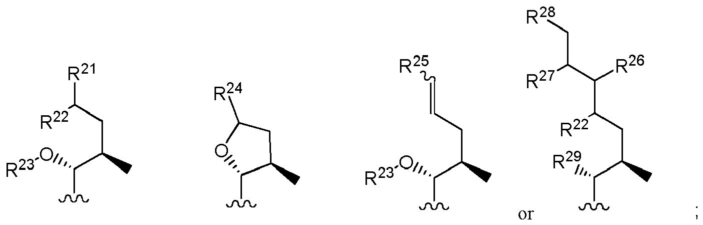
R² is C₁-C₆ alkyl, C₃-C₆ cycloalkyl, or hydroxy-C₁-C₆ alkyl;
R⁴, R⁵ and R¹¹ are independently C₁-C₆ alkyl or C₃-C₆ cycloalkyl;
R⁸ is C₁-C₆ alkyl, hydroxy-C₁-C₆ alkyl, or amino-C₁-C₆ alkyl;
R¹², R¹³, R¹⁴ and R¹⁵ are independently H or methyl;
R¹⁷ is -CHCHCH₂CH₂-, -CHCHCH₂C(O)-, -(CH₂)₄- or -(CH₂)₃C(O)-;
R¹⁸ is OH, O-C₁-C₆ alkyl, OC(O)-C₁-C₆ alkyl, NH₂, NH-C₁-C₆ alkyl or NHC(O)-C₁-C₆ alkyl;
R²¹ is H, methyl, C₁-C₆ alkyl, C₂-C₆ alkenyl, C₃-C₆ cycloalkyl or phenyl;
R²³ is H or C₁-C₆ alkyl;
R²⁴ is H, halogen, C₁-C₆ alkyl, C₂-C₆ alkenyl, -NHR²¹, hydroxy or C₁-C₆ alkoxy;
R²⁵ is C₁-C₆ alkyl;
wherein R²¹, R²⁴ and R²⁵ are optionally substituted one or more times with -OH, -CHO, or -CO₂H; and
wherein any two adjacent carbon atoms of R²¹, R²², R²⁴ and R²⁵, together with an oxygen atom, may form an epoxide;
R²², R²⁶, R²⁷, R²⁸ and R²⁹ are independently H, halogen, cyano, nitro, methyl, C₁-C₆ alkyl, C₂-C₆ alkenyl, -OR³⁰, -C(O)R³⁰, -OC(O)R³⁰, -C(O)OR³⁰, -S(O)R³⁰, -SO₂R³⁰, -SO₃R³⁰, -OSO₂R³⁰, -OSO₃R³⁰, -PO₂R³⁰R³¹, -OPO₂R³⁰R³¹, -PO₃R³⁰R³¹, -OPO₃ R³⁰R³¹, -N(R³⁰)R³¹, -C(O)N(R³⁰)R³¹, - C(O)NR³⁰NR³¹SO₂R³², -C(O)NR³⁰SO₂R³², -C(O)NR³⁰CN, -SO₂N(R³⁰)R³¹, -SO₂N(R³⁰)R³¹, NR³²C(O)R³⁰, -NR³²C(O)OR³⁰ or -NR³²C(O)N(R³⁰)R³¹; or R²⁶ and R²⁷ are together -O-; or R²⁸ and R²⁷ together, or R²² and R²⁶ together, are independently -O-; or R²² and R²⁹ together are -O-; or R²⁸, together with the carbon to which it is bonded, is -C(=O)R³⁰, -CO₂R³⁰, -CH₂OR³⁰, - CH₂OC(O)R³⁰, -CH(OR³⁰)², -C(O)N(R³⁰R³¹), -C(=NR³¹)R³⁰, -C(=NOR³¹)R³⁰, or -C(=NNR³¹)R³⁰; provided that at least one pair of R²⁷ and R²⁸, R²⁶ and R²⁷, or R²² and R²⁶ do not form a ring; and
R³⁰, R³¹ and R³² are each independently -H or an optionally substituted aliphatic, cycloaliphatic, benzyl, or aryl, or -N(R³⁰)R³¹ together is an optionally substituted heterocyclic group, or -CH(OR³⁰)2 together is a cyclic acetal group.

2. A cyclosporine H derivative as defined in claim 1, cyclosporine H or dihydro cyclosporine H for use in treating a subject suffering from an eosinophil-associated disease by administering to the subject a therapeutically effective amount of the said cyclosporine H derivative, cyclosporine H or dihydro cyclosporine H.

3. Cyclosporine H or dihydro cyclosporine H for use as claimed in claim 2, wherein said eosinophil-associated disease is an immune disorder or an allergic disorder.

4. Cyclosporine H or dihydro cyclosporine H for use as claimed in claim 3, wherein said immune disorder is selected from the group consisting of eczema and dermatitis, and psoriasis.

5. Cyclosporine H or dihydro cyclosporine H for use as claimed in claim 3, wherein said allergic disorder is selected from the group consisting of asthma, hay fever, hypereosinophilic syndrome, or an eosinophil-associated gastrointestinal disorder.

6. Cyclosporine H or dihydro cyclosporine H for use as claimed in claim 2, wherein said eosinophil-associated disease is selected from the group consisting of eosinophilic gastroenteritis, allergic colitis, eosinophilic esophagitis, atopic dermatitis, inflammatory bowel disease and gastrointestinal reflux disease.

7. A cyclosporine H derivative as defined in claim 1 for use in inhibiting the activity of a formyl peptide receptor in a subject by administering to said subject an effective amount of said cyclosporine H derivative such that eosinophil mobilization in said subject is inhibited.

8. A cyclosporine H derivative as defined in claim 1, cyclosporine H or dihydro cyclosporine H for use in decreasing eosinophil mobilization in a subject by administering to said subject an effective amount of the said cyclosporine H derivative, cyclosporine H or dihydro cyclosporine H

9. A cyclosporine H derivative as defined in claim 1, cyclosporine H or dihydro cyclosporine H for use in decreasing the level of circulating eosinophils in a subject by administering to said subject said cyclosporine H derivative, cyclosporine H or dihydro cyclosporine H in an amount sufficient to decrease said level of circulating eosinophils in said subject.

10. A cyclosporine H derivative as defined in claim 1, cyclosporine H or dihydro cyclosporine H for use in decreasing the accumulation of eosinophils at sites associated with a disease related to increased eosinophilic function or decreasing localized eosinophil cell numbers in a subject by administering to said subject a therapeutically effective amount of the said cyclosporine H derivative, cyclosporine H or dihydro cyclosporine H.

11. A formyl peptide receptor (FPR) antagonist, cyclosporine H derivative as defined in claim 1, cyclosporine H or dihydro cyclosporine H for use in treating a subject suffering from a chronic obstructive pulmonary disease (COPD) by administering a therapeutically effective amount of said formyl peptide receptor (FPR) antagonist, cyclosporine H derivative, cyclosporine H or dihydro cyclosporine H.

12. A formyl peptide receptor (FPR) antagonist as claimed in claim 11 wherein the FPR antagonist is CsH or dihydroCsH.

13. A formyl peptide receptor (FPR) antagonist, cyclosporine H derivative as defined in claim 1, cyclosporine H or dihydro cyclosporine H for use in treating a subject suffering from emphysema by administering a therapeutically effective amount of said formyl peptide receptor (FPR) antagonist, cyclosporine H derivative, cyclosporine H or dihydro cyclosporine H.

14. A formyl peptide receptor (FPR) antagonist, cyclosporine H derivative as defined in claim 1, cyclosporine H or dihydro cyclosporine H for use in treating a subject suffering from an inflammatory disease by administering a therapeutically effective amount of said formyl peptide receptor (FPR) antagonist, cyclosporine H derivative, cyclosporine H or dihydro cyclosporine H.

15. A formyl peptide receptor (FPR) antagonist, cyclosporine H derivative as defined in claim 1, cyclosporine H or dihydro cyclosporine H wherein the FPR antagonist is dihydrocyclosporine H (dihydroCsH).

16. Dihydrocyclosporine H for use in a treating a subject suffering from an inflammatory disease by administering a therapeutically effective amount of dihydrocyclosporine H.

17. Dihydrocyclosporine H for use as claimed in claim 16 in treating an inflammatory disease selected from the group consisting of acute lung inflammation, chronic lung inflammation, COPD, emphysema, inflammation of the gastrointestinal tract, acute skin inflammation, acne, atopic dermatitis, eczema, neutrophilic dermatosis, rosacea, psoriasis, peritonitis, ischemic reperfusion injury, neutrophil disorder, cystic fibrosis, and diseases caused by or associated with detrimental, exaggerated, or inappropriate neutrophil activation, or a combination thereof.

18. Dihydrocyclosporine H for use as claimed in claim 17 in treating inflammatory bowel disease (IBD) or Crohn's disease.

19. Dihydrocyclosporine H for use as claimed in claim 16 in treating a disorder selected from the group consisting of chronic granulomatous disease, Chediak-Higashi syndrome (CHS), leukocyte adhesion deficiency, hyper IgE syndrome (Jobs syndrome), and other neutrophil-mediated inflammation.

20. Dihydrocyclosporine H for use as claimed in claim 17, wherein the disease with detrimental or inappropriate neutrophil activation is cystic fibrosis, inflammatory bowel disease (IBD) or Crohn's disease.

## Patentansprüche

1. Cyclosporin-H-Derivat der Formel I: 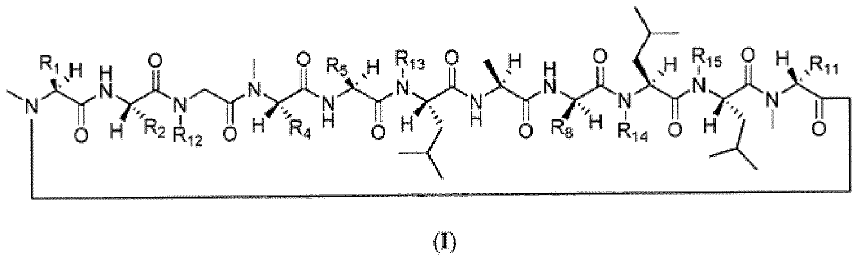 und pharmazeutisch verträgliche Salze und Solvate davon, wobei
R¹CH(OH)CH(CH₃)CH₂CH₂R¹⁷R¹⁸, 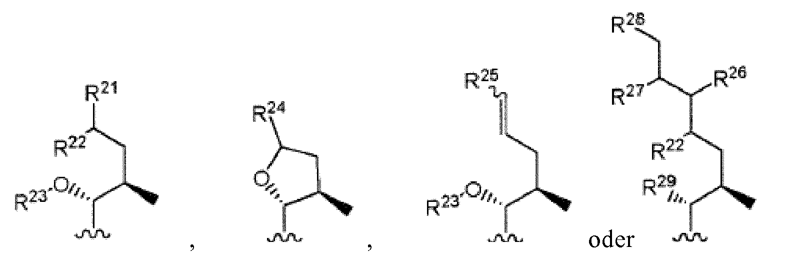 ist;
R² für C₁-C₆-Alkyl, C₃-C₆-Cycloalkyl oder Hydroxy-C₁-C₆-alkyl steht;
R⁴, R⁵ und R¹¹ unabhängig voneinander C₁-C₆-Alkyl oder C₃-C₆-Cycloalkyl sind;
R⁸ für C₁-C₆-Alkyl, Hydroxy-C₁-C₆-alkyl oder Amino-C₁-C₆-alkyl steht;
R¹², R¹³, R¹⁴ und R¹⁵ unabhängig voneinander H oder Methyl sind;
R¹⁷ -CHCHCH₂CH₂-, -CHCHCH₂C(O)-, -(CH₂)₄- oder -(CH₂)₃C(O)- ist;
R¹⁸ für OH, O-C₁-C₆-Alkyl, OC(O)-C₁-C₆-Alkyl, NH₂, NH-C₁-C₆-Alkyl oder NHC(O)-C₁-C₆-Alkyl steht;
R²¹ für H, Methyl, C₁-C₆-Alkyl, C₂-C₆-Alkenyl, C₃-C₆-Cycloalkyl oder Phenyl steht;
R²³ für H oder C₁-C₆-Alkyl steht;
R²⁴ für H, Halogen, C₁-C₆-Alkyl, C₂-C₆-Alkenyl, -NHR²¹, Hydroxy oder C₁-C₆-Alkoxy steht;
R²⁵ für C₁-C₆-Alkyl steht;
wobei R²¹, R²⁴ und R²⁵ gegebenenfalls ein- oder mehrfach mit -OH, -CHO oder -CO₂H substituiert sind; und
wobei jeweils zwei benachbarte Kohlenstoffatome von R²¹, R²², R²⁴ und R²⁵, zusammen mit einem Sauerstoffatom, eine Epoxygruppe bilden können;
R²², R²⁶, R²⁷, R²⁸ und R²⁹ unabhängig voneinander H, Halogen, Cyano, Nitro, Methyl, C₁-C₆-Alkyl, C₂-C₆-Alkenyl, -OR³⁰, -C(O)R³⁰, -OC(O)R³⁰, -C(O)OR³⁰, -S(O)R³⁰, -SO₂R³⁰, -SO₃R³⁰, -OSO₂R³⁰, -OSO₃R³⁰, -PO₂R³⁰R³¹, -OPO₂R³⁰R³¹, -PO₃R³⁰R³¹, -OPO₃R³⁰R³¹, -N(R³⁰)R³¹, -C(O)N(R³⁰)R³¹, -C(O)NR³⁰NR³¹SO₂R³², -C(O)NR³⁰SO₂R³², -C(O)NR³⁰CN, -SO₂N(R³⁰)R³¹, -NR³²C(O)R³⁰, -NR³²C(O)OR³⁰ oder -NR³²C(O)N(R³⁰)R³¹ sind; oder R²⁶ und R²⁷ zusammen -O- sind; oder R²⁷ und R²⁸ zusammen oder R²² und R²⁶ zusammen unabhängig voneinander -O- sind; oder R²² und R²⁹ zusammen -O- sind; oder R²⁸, zusammen mit dem Kohlenstoffatom, an das er gebunden ist, - C(=O)R³⁰, -CO₂R³⁰, -CH₂OR³⁰, -CH₂OC(O)R³⁰, -CH(OR³⁰)2, -C(O)N(R³⁰R³¹), -C(=NR³¹)R³⁰, -C(=NOR³¹)R³⁰ oder -C(=NNR³¹)R³⁰ ist; mit der Maßgabe, dass mindestens ein Paar von R²⁷ und R²⁸, R²⁶ und R²⁷ oder R²² und
R²⁶ keinen Ring bildet; und
R³⁰, R³¹ und R³² jeweils unabhängig voneinander -H oder ein gegebenenfalls substituierter aliphatischer, cycloaliphatischer Rest, Benzyl- oder Arylrest sind, oder -N(R³⁰)R³¹ zusammen ein gegebenenfalls substituierter heterocyclischer Rest ist, oder -CH(OR³⁰)₂ zusammen ein cyclischer Acetalrest ist.

2. Ein Cyclosporin-H-Derivat wie in Anspruch 1 definiert, Cyclosporin H oder Dihydrocyclosporin H zur Verwendung bei der Behandlung eines Subjektes, das an einer mit Eosinophilen in Zusammenhang stehenden Erkrankung leidet, durch Verabreichung einer therapeutisch wirksamen Menge von dem Cyclosporin-H-Derivat, Cyclosporin H oder Dihydrocyclosporin H an das Subjekt.

3. Cyclosporin H oder Dihydrocyclosporin H zur Verwendung gemäß Anspruch 2, wobei die mit Eosinophilen in Zusammenhang stehende Erkrankung eine Immunerkrankung oder eine allergische Erkrankung ist.

4. Cyclosporin H oder Dihydrocyclosporin H zur Verwendung gemäß Anspruch 3, wobei die Immunerkrankung ausgewählt ist aus Ekzem und Dermatitis, und Psoriasis.

5. Cyclosporin H oder Dihydrocyclosporin H zur Verwendung gemäß Anspruch 3, wobei die allergische Erkrankung ausgewählt ist aus Asthma, Heuschnupfen, hypereosinophilem Syndrom oder einer mit Eosinophilen in Zusammenhang stehenden gastrointestinalen Erkrankung.

6. Cyclosporin H oder Dihydrocyclosporin H zur Verwendung gemäß Anspruch 2, wobei die mit Eosinophilen in Zusammenhang stehende Erkrankung ausgewählt ist aus eosinophiler Gastroenteritis, allergischer Colitis, eosinophiler Ösophagitis, atopischer Dermatitis, entzündlicher Darmerkrankung und gastrointestinaler Refluxkrankheit.

7. Ein Cyclosporin-H-Derivat wie in Anspruch 1 definiert zur Verwendung bei der Hemmung der Aktivität eines Formyl-Peptidrezeptors bei einem Subjekt durch Verabreichung einer wirksamen Menge des Cyclosporin-H-Derivats an das Subjekt, sodass die Mobilisierung von Eosonophilen bei dem Subjekt gehemmt wird.

8. Ein Cyclosporin-H-Derivat wie in Anspruch 1 definiert, Cyclosporin H oder Dihydrocyclosporin H zur Verwendung bei der Verringerung der Mobilisierung von Eosonophilen bei einem Subjekt durch Verabreichung einer wirksamen Menge von dem Cyclosporin-H-Derivat, Cyclosporin H oder Dihydrocyclosporin H an das Subjekt.

9. Ein Cyclosporin-H-Derivat wie in Anspruch 1 definiert, Cyclosporin H oder Dihydrocyclosporin H zur Verwendung bei der Verringerung der Zahl zirkulierender Eosinophiler bei einem Subjekt durch Verabreichung von dem Cyclosporin-H-Derivat, Cyclosporin H oder Dihydrocyclosporin H an das Subjekt in einer Menge, die ausreichend ist, um die Zahl zirkulierender Eosinophiler bei dem Subjekt zu reduzieren.

10. Ein Cyclosporin-H-Derivat wie in Anspruch 1 definiert, Cyclosporin H oder Dihydrocyclosporin H zur Verwendung bei der Verringerung der Akkumulation von Eosinophilen an Stellen, die mit einer Erkrankung in Zusammenhang stehen, die mit erhöhter Eosinophilenfunktion verbunden ist, oder bei der Herabsetzung der Zahl lokalisierter eosinophiler Zellen bei einem Subjekt durch Verabreichung einer therapeutisch wirksamen Menge von dem Cyclosporin-H-Derivat, Cyclosporin H oder Dihydrocyclosporin H an das Subjekt.

11. Ein Antagonist des Formyl-Peptidrezeptors (FPR), Cyclosporin-H-Derivat wie in Anspruch 1 definiert, Cyclosporin H oder Dihydrocyclosporin H zur Verwendung bei der Behandlung eines Subjektes, das an chronisch obstruktiver Lungenerkrankung (COPD) leidet, durch Verabreichung einer therapeutisch wirksamen Menge von dem Antagonisten des Formyl-Peptidrezeptors (FPR), Cyclosporin-H-Derivat, Cyclosporin H oder Dihydrocyclosporin H.

12. Ein Antagonist des Formyl-Peptidrezeptors (FPR) gemäß Anspruch 11, wobei der FPR-Antagonist CsH oder DihydroCsH ist.

13. Ein Antagonist des Formyl-Peptidrezeptors (FPR), Cyclosporin-H-Derivat wie in Anspruch 1 definiert, Cyclosporin H oder Dihydrocyclosporin H zur Verwendung bei der Behandlung eines Subjektes, das an einem Emphysem leidet, durch Verabreichung einer therapeutisch wirksamen Menge von dem Antagonisten des Formyl-Peptidrezeptors (FPR), Cyclosporin-H-Derivat, Cyclosporin H oder Dihydrocyclosporin H.

14. Ein Antagonist des Formyl-Peptidrezeptors (FPR), Cyclosporin-H-Derivat wie in Anspruch 1 definiert, Cyclosporin H oder Dihydrocyclosporin H zur Verwendung bei der Behandlung eines Subjektes, das an einer entzündlichen Erkrankung leidet, durch Verabreichung einer therapeutisch wirksamen Menge von dem Antagonisten des Formyl-Peptidrezeptors (FPR), Cyclosporin-H-Derivat, Cyclosporin H oder Dihydrocyclosporin H.

15. Ein Antagonist des Formyl-Peptidrezeptors (FPR), Cyclosporin-H-Derivat wie in Anspruch 1 definiert, Cyclosporin H oder Dihydrocyclosporin H, wobei der FPR-Antagonist Dihydrocyclosporin H (DihydroCsH) ist.

16. Dihydrocyclosporin H zur Verwendung bei der Behandlung eines Subjektes, das an einer entzündlichen Erkrankung leidet, durch Verabreichung einer therapeutisch wirksamen Menge von Dihydrocyclosporin H.

17. Dihydrocyclosporin H zur Verwendung gemäß Anspruch 16 bei der Behandlung einer entzündlichen Erkrankung, ausgewählt aus akuter Lungenentzündung, chronischer Lungenentzündung, COPD, Emphysem, Entzündung des Magen-Darm-Traktes, akuter Hautentzündung, Akne, atopischer Dermatitis, Ekzem, neutrophiler Dermatose, Rosacea, Psoriasis, Peritonitis ischämischer Reperfusionsschädigung, neutrophiler Erkrankung, Mukoviszidose und Erkrankungen, ausgelöst durch schädliche, überhöhte oder ungünstige Aktivierung von Neutrophilen oder damit verbunden, oder einer Kombination davon.

18. Dihydrocyclosporin H zur Verwendung gemäß Anspruch 17 bei der Behandlung von entzündlicher Darmerkrankung (IBD) oder Morbus Crohn.

19. Dihydrocyclosporin H zur Verwendung gemäß Anspruch 16 bei der Behandlung einer Erkrankung, ausgewählt aus chronischer granulomatöser Erkrankung, Chediak-Higashi-Syndrom (CHS), Leukozytenadhäsionsmangel, Hyper-IgE-Syndrom (Job-Syndrom) und anderen durch Neutrophile vermittelten Entzündungen.

20. Dihydrocyclosporin H zur Verwendung gemäß Anspruch 17, wobei die Erkrankung mit schädlicher oder ungünstiger Aktivierung von Neutrophilen Mukoviszidose, entzündliche Darmerkrankung (IBD) oder Morbus Crohn ist.

## Revendications

1. Dérivé de cyclosporine H de formule I : 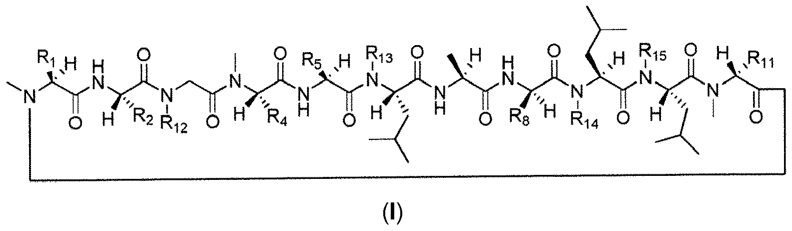 et sels et solvates pharmaceutiquement acceptables de celui-ci, dans lesquels
R¹ est CH(OH)CH(CH₃)CH₂CH₂R¹⁷R¹⁸, 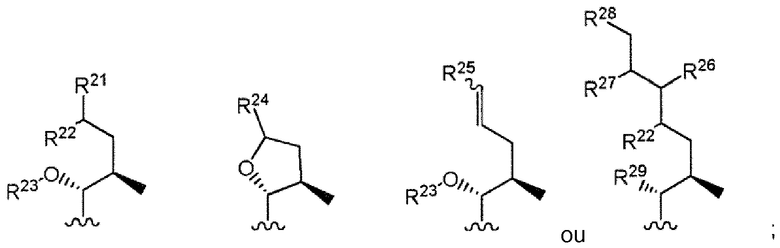
R² est alkyle en C₁-C₆, cycloalkyle en C₃-C₆ ou hydroxyalkyle en C₁-C₆ ;
R⁴, R⁵ et R¹¹ sont indépendamment alkyle en C₁-C₆ ou cycloalkyle en C₃-C₆;
R⁸ est alkyle en C₁-C₆, hydroxyalkyle en C₁-C₆ ou aminoalkyle en C₁-C₆;
R¹², R¹³, R¹⁴ et R¹⁵ sont indépendamment H ou méthyle ;
R¹⁷ est -CHCHCH₂CH₂-, -CHCHCH₂C(O)-, -(CH₂)₄- ou -(CH₂)₃C(O)- ;
R¹⁸ est OH, O-alkyle en C₁-C₆, OC(O)-alkyle en C₁-C₆, NH₂, NH-alkyle en C₁-C₆ ou NHC(O)-alkyle en C₁-C₆ ;
R²¹ est H, méthyle, alkyle en C₁-C₆, alcényle en C₂-C₆, cycloalkyle en C₃-C₆ ou phényle ;
R²³ est H ou alkyle en C₁-C₆ ;
R²⁴ est H, halogène, alkyle en C₁-C₆, alcényle en C₂-C₆, -NHR²¹, hydroxy ou alcoxy en C₁-C₆ ;
R²⁵ est alkyle en C₁-C₆ ;
dans lesquels R²¹, R²⁴ et R²⁵ sont éventuellement substitués une ou plusieurs fois par -OH, -CHO ou -CO₂H ; et
dans lesquels toute paire d'atomes de carbone adjacents de R²¹, R²², R²⁴ et R²⁵, conjointement avec un atome d'oxygène, peut former un époxyde ;
R²², R²⁶, R²⁷, R²⁸ et R²⁹ sont indépendamment H, halogène, cyano, nitro, méthyle, alkyle en C₁-C₆, alcényle en C₂-C₆, -OR³⁰, -C(O)R³⁰, -OC(O)R³⁰, - C(O)OR³⁰, -S(O)R³⁰, -SO₂R³⁰, -SO₃R³⁰, -OSO₂R³⁰, -OSO₃R³⁰, -PO₂R³⁰R³¹, - OPO₂R³⁰R³¹, -PO₃R³⁰R³¹, -OPO₃R³⁰R³¹, -N(R³⁰)R³¹, -C(O)N(R³⁰)R³¹, - C(O)NR³⁰NR³¹SO₂R³², -C(O)NR³⁰SO₂R³², -C(O)NR³⁰CN, -SO₂N(R³⁰)R³¹, - SO₂N(R³⁰)R³¹, -NR³²C(O)R³⁰, -NR³²C(O)OR³⁰ ou -NR³²C(O)N(R³⁰)R³¹ ; ou R²⁶ et R²⁷ sont ensemble -O- ; ou R²⁸ et R²⁷ ensemble, ou R²² et R²⁶ ensemble, sont indépendamment -O- ; ou R²² et R²⁹ ensemble sont -O- ; ou R²⁸, conjointement avec l'atome de carbone auquel il est rattaché, est -C(=O)R³⁰, -CO₂R³⁰, - CH₂OR³⁰, -CH₂OC(O)R³⁰, -CH(OR³⁰)₂, -C(O)N(R³⁰R³¹), -C(=NR³¹)R³⁰, - C(=NOR³¹)R³⁰ ou -C(=NNR³¹)R³⁰ ; à condition qu'au moins une paire parmi R²⁷ et R²⁸, R²⁶ et R²⁷ ou R²² et R²⁶ ne forme pas de cycle ; et
R³⁰ , R³¹ et R³² sont chacun indépendamment -H ou un groupe aliphatique, cycloaliphatique, benzyle ou aryle éventuellement substitué ou - N(R³⁰)R³¹ ensemble est un groupe hétérocyclique éventuellement substitué, ou -CH(OR³⁰)₂ ensemble est un groupe acétal cyclique.

2. Dérivé de cyclosporine H selon la revendication 1, cyclosporine H ou dihydrocyclosporine H pour une utilisation dans le traitement d'un sujet souffrant d'une maladie associée aux éosinophiles par l'administration au sujet d'une quantité thérapeutiquement efficace desdits dérivé de cyclosporine H, cyclosporine H ou dihydrocyclosporine H.

3. Cyclosporine H ou dihydrocyclosporine H pour une utilisation selon la revendication 2, dans lesquelles ladite maladie associée aux éosinophiles est un trouble immunitaire ou un trouble allergique.

4. Cyclosporine H ou dihydrocyclosporine H pour une utilisation selon la revendication 3, dans lesquelles ledit trouble immunitaire est sélectionné dans le groupe constitué par l'eczéma et la dermatite, et le psoriasis.

5. Cyclosporine H ou dihydrocyclosporine H pour une utilisation selon la revendication 3, dans lesquelles ledit trouble allergique est sélectionné dans le groupe constitué par l'asthme, la rhinite allergique saisonnière, le syndrome d'hyperéosinophilie ou un trouble gastro-intestinal associé aux éosinophiles.

6. Cyclosporine H ou dihydrocyclosporine H pour une utilisation selon la revendication 2, dans lesquelles ladite maladie associée aux éosinophiles est sélectionnée dans le groupe constitué par la gastroentérite à éosinophiles, la colite allergique, l'oesophagite à éosinophiles, la dermatite atopique, la maladie intestinale inflammatoire et la maladie du reflux gastro-oesophagien.

7. Dérivé de cyclosporine H selon la revendication 1 pour une utilisation dans l'inhibition de l'activité d'un récepteur des peptides formylés chez un sujet par l'administration audit sujet d'une quantité efficace dudit dérivé de cyclosporine H de telle sorte que la mobilisation des éosinophiles chez ledit sujet est inhibée.

8. Dérivé de cyclosporine H selon la revendication 1, cyclosporine H ou dihydrocyclosporine H pour une utilisation dans la diminution de la mobilisation des éosinophiles chez un sujet par l'administration audit sujet d'une quantité efficace desdits dérivé de cyclosporine H, cyclosporine H ou dihydrocyclosporine H.

9. Dérivé de cyclosporine H selon la revendication 1, cyclosporine H ou dihydrocyclosporine H pour une utilisation dans la réduction du taux d'éosinophiles circulants chez un sujet par l'administration audit sujet desdits dérivé de cyclosporine H, cyclosporine H ou dihydrocyclosporine H en une quantité suffisante pour réduire ledit taux d'éosinophiles circulants chez ledit sujet.

10. Dérivé de cyclosporine H selon la revendication 1, cyclosporine H ou dihydrocyclosporine H pour une utilisation dans la réduction de l'accumulation des éosinophiles sur des sites associés à une maladie liée à une fonction accrue des éosinophiles ou la réduction du nombre de cellules d'éosinophiles localisées chez un sujet par l'administration audit sujet d'une quantité thérapeutiquement efficace desdits dérivé de cyclosporine H, cyclosporine H ou dihydrocyclosporine H.

11. Antagoniste des récepteurs des peptides formylés (FPR), dérivé de cyclosporine H selon la revendication 1, cyclosporine H ou dihydrocyclosporine H pour une utilisation dans le traitement d'un sujet souffrant d'une maladie pulmonaire obstructive chronique (MPOC) par l'administration audit sujet d'une quantité thérapeutiquement efficace desdits antagoniste des récepteurs des peptides formylés (FPR), dérivé de cyclosporine H, cyclosporine H ou dihydrocyclosporine H.

12. Antagoniste des récepteurs des peptides formylés (FPR) selon la revendication 11, dans lequel l'antagoniste des FPR est la CsH ou la dihydroCsH.

13. Antagoniste des récepteurs des peptides formylés (FPR), dérivé de cyclosporine H selon la revendication 1, cyclosporine H ou dihydrocyclosporine H pour une utilisation dans le traitement d'un sujet souffrant d'un emphysème par l'administration audit sujet d'une quantité thérapeutiquement efficace desdits antagoniste des récepteurs des peptides formylés (FPR), dérivé de cyclosporine H, cyclosporine H ou dihydrocyclosporine H.

14. Antagoniste des récepteurs des peptides formylés (FPR), dérivé de cyclosporine H selon la revendication 1, cyclosporine H ou dihydrocyclosporine H pour une utilisation dans le traitement d'un sujet souffrant d'une maladie inflammatoire par l'administration audit sujet d'une quantité thérapeutiquement efficace desdits antagoniste des récepteurs des peptides formylés (FPR), dérivé de cyclosporine H, cyclosporine H ou dihydrocyclosporine H.

15. Antagoniste des récepteurs des peptides formylés (FPR), dérivé de cyclosporine H selon la revendication 1, cyclosporine H ou dihydrocyclosporine H, dans lesquels l'antagoniste des FPR est la dihydrocyclosporine H (dihydroCsH).

16. Dihydrocyclosporine H pour une utilisation dans le traitement d'un sujet souffrant d'une maladie inflammatoire par l'administration d'une quantité thérapeutiquement efficace de dihydrocyclosporine H.

17. Dihydrocyclosporine H pour une utilisation selon la revendication 16 dans le traitement d'une maladie inflammatoire sélectionnée dans le groupe constitué par l'inflammation pulmonaire aiguë, l'inflammation pulmonaire chronique, la MPOC, l'emphysème, une inflammation du tube digestif, une inflammation cutanée aiguë, l'acné, la dermatite atopique, l'eczéma, la dermatose neutrophile, la couperose, le psoriasis, une péritonite, une lésion ischémique de reperfusion, un trouble des neutrophiles, la fibrose kystique, et les maladies causées par ou associées à une activation préjudiciable, exagérée ou inappropriée des neutrophiles ou une association de celles-ci.

18. Dihydrocyclosporine H pour une utilisation selon la revendication 17 dans le traitement de la maladie intestinale inflammatoire (MII) ou de la maladie de Crohn.

19. Dihydrocyclosporine H pour une utilisation selon la revendication 16 dans le traitement d'un trouble sélectionné dans le groupe constitué par la granulomatose chronique, le syndrome de Chédiak-Higashi (SCH), un déficit d'adhésion leucocytaire, le syndrome d'hypergammaglobulinémie IgE (syndrome de Job) et d'autres inflammations médiées par les neutrophiles.

20. Dihydrocyclosporine H pour une utilisation selon la revendication 17, dans laquelle la maladie présentant une activation préjudiciable ou inappropriée des neutrophiles est la fibrose kystique, la maladie intestinale inflammatoire (MII) ou la maladie de Crohn.
